# EUROPEAN PATENT APPLICATION

(11) **EP 4 635 451 A1**
(43) Date of publication of application: **22.10.2025**
(21) Application number: 25170357.5
(22) Date of filing: 14.04.2025
(51) Int. Cl.: A61F 2/24, A61F 2/95

(54) **TRANSCATHETER DEVICES AND METHODS**

(30) Priority: 21.04.2024 US 202463636795 P; 04.04.2025 US 202519170596
(71) Applicant: Medtronic, Inc., Minneapolis, Minnesota 55432 (US)
(72) Inventor: Duffy, Niall F., Galway (IE); Haribabu, Sumeeyae, Galway (IE); McLoone, Maryrose, Galway (IE); Mallon, Sarah, Galway (IE)
(74) Representative: Zimmermann & Partner Patentanwälte mbB

(57) **Abstract**

Transcatheter devices comprise a shaft comprising a proximal portion and a distal portion. The transcatheter devices further comprise a handle device coupled to the proximal portion of the shaft. The handle device comprises a first actuator. The transcatheter devices further comprise a distal tip coupled to the distal portion of the shaft. The transcatheter devices further comprise a severing device comprising a proximal portion pivotally mounted to the distal tip. The severing device configured to be pivoted from a loaded state to a deployed state. Methods include moving a severing device from a loaded state to a deployed state. Methods further include severing at least one leaflet with the severing device in the deployed state and radially expanding the expandable stent frame.

## Description

### CROSS-REFERENCE TO RELATED APPLICATIONS

This application claims the benefit of U.S. Provisional Patent Application Serial No. 63/636,795, filed April 21, 2024, and U.S. Patent Application Serial No. 19/170,596, filed April 4, 2025, the entire contents of which are incorporated herein by reference.

### FIELD

The present disclosure relates generally to transcatheter devices and methods, and more particularly to transcatheter devices including a severing device, and methods of replacing and/or managing leaflets of a defective heart valve with a transcatheter device including a severing device.

### BACKGROUND

A human heart includes four heart valves that determine the pathway of blood flow through the heart: the mitral valve, the tricuspid valve, the aortic valve, and the pulmonary valve. The mitral and tricuspid valves are atrio-ventricular valves, which are between the atria and the ventricles, while the aortic and pulmonary valves are semilunar valves, which are in the arteries leaving the heart. Ideally, native leaflets of a heart valve move apart from each other when the valve is in an open position and meet or "coapt" when the valve is in a closed position. Problems that may develop with valves include stenosis in which a valve does not open properly, and/or insufficiency or regurgitation in which a valve does not close properly. Stenosis and insufficiency may occur concomitantly in the same valve. The effects of valvular dysfunction vary, with regurgitation or backflow typically having relatively severe physiological consequences to the patient.

Diseased or otherwise deficient heart valves can be repaired or replaced using a variety of different types of heart valve surgeries. One conventional technique involves an open-heart surgical approach that is conducted under general anesthesia, during which the heart is stopped, and blood flow is controlled by a heart-lung bypass machine.

More recently, minimally invasive approaches have been developed to facilitate catheter-based implantation of a prosthetic heart valve or prosthesis on the beating heart, intending to obviate the need for the use of classical sternotomy and cardiopulmonary bypass. In general terms, an expandable prosthetic valve is compressed about or within a catheter, inserted inside a body lumen of the patient, such as the femoral artery, and delivered to a desired location in the heart.

However, over time these prosthetic heart valves may also begin to become damaged or diseased, such as for example, stenosis. Accordingly, the diseased or damaged prosthetic heart valve may need replaced. Similar to that described above, catheter-based approaches have also been used to repair or replace the damaged prosthetic heart valves.

In light of the above, a need exists for a transcatheter device that can manage the leaflets of a heart valve during catheter-based implantations of prosthetic heart valves.

### SUMMARY

The following presents a simplified summary of the disclosure to provide a basic understanding of some aspects described in the detailed description.

Features of the present disclosure provide a transcatheter device having a severing device to facilitate the replacement of a damaged and/or diseased heart valve by managing one or more leaflets of the defective heart valve. Providing a transcatheter device with a severing device that can manage the leaflets of the defective heart valve can allow a clinician to replace the defective heart valve while preventing obstructions that may be caused by the leaflets.

In aspects, transcatheter devices comprise a shaft comprising a proximal portion and a distal portion. The transcatheter devices further comprise a handle device coupled to the proximal portion of the shaft. The handle device comprises a first actuator. The transcatheter devices still further comprise a distal tip coupled to the distal portion of the shaft. The transcatheter devices still further comprise a sheath disposed around the shaft and configured to translate relative to the distal tip. The transcatheter devices can still further comprise a severing device comprising a proximal portion pivotally mounted to the distal tip. The severing device is configured to be pivoted from a loaded state to a deployed state. The transcatheter devices can further comprise a first actuator configured to be activated to move the severing device from the loaded state to the deployed state.

In further aspects, methods of replacing a heart valve comprise distally advancing an expandable stent frame of a transcatheter device in a contracted orientation into the heart valve. The methods further comprise aligning a portion of the transcatheter device with leaflets of the heart valve. The methods further comprise moving a severing device of the transcatheter device from a loaded state to a deployed state. The methods still further comprise severing at least one of the leaflets with the severing device in the deployed state. The methods still further comprise radially expanding the expandable stent frame after severing at least one of the leaflets.

Additional features and advantages of the aspects disclosed herein will be set forth in the detailed description that follows, and in part will be clear to those skilled in the art from that description or recognized by practicing the aspects described herein, including the detailed description which follows, the claims, as well as the appended drawings. It is to be understood that both the foregoing general description and the following detailed description present aspects intended to provide an overview or framework for understanding the nature and character of the aspects disclosed herein. The accompanying drawings are included to provide further understanding and are incorporated into and constitute a part of this specification. The drawings illustrate various aspects of the disclosure, and together with the description explain the principles and operations thereof.

### BRIEF DESCRIPTION OF THE DRAWINGS

These and other features, aspects and advantages are better understood when the following detailed description is read with reference to the accompanying drawings, in which:
FIG. 1 is a schematic view of an exemplary transcatheter device in accordance with aspects of the present disclosure;
FIG. 2 is an enlarged view of the distal end portion of the exemplary transcatheter device taken at view 2 of FIG. 1;
FIG. 3 is a schematic cross-sectional view of an embodiment of the distal end portion of the transcatheter device taken at line 3-3 of FIG. 2 showing a severing device in a loaded state;
FIG. 4 is a schematic cross-sectional view of a portion of the transcatheter device of FIG. 1 taken at line 4-4 illustrating an expandable stent frame in a contracted orientation;
FIG. 5 is a schematic cross-sectional view of the distal end portion of FIG. 3 showing the severing device in the deployed state;
FIG. 6 is a schematic cross-sectional view of the portion of the transcatheter device of FIG. 4, showing the expandable stent frame in a partially expanded orientation;
FIG. 7 is a schematic cross-sectional view of another embodiment of the distal end portion of the transcatheter device taken at line 3-3 of FIG. 2 showing a severing device in a loaded state;
FIG. 8 is a schematic cross-sectional view of another embodiment of a portion of the transcatheter device of FIG. 1 taken at line 4-4 illustrating an expandable stent frame in a contracted orientation;
FIG. 9 is a schematic cross-sectional view of the distal end portion of FIG. 7 showing the severing device in the deployed state;
FIG. 10 is a schematic cross-sectional view of the portion of the transcatheter device of FIG. 8, showing the expandable stent frame in a partially expanded orientation;
FIG. 11 is a schematic exploded view of an embodiment of the transcatheter device in accordance with aspects of the disclosure;
FIG. 12 is a schematic side view of an exemplary expandable stent frame that can be employed with the transcatheter device;
FIG. 13 is a schematic cross-sectional view of the exemplary expandable stent frame taken at line 13-13 of FIG. 12 showing leaflets coupled to the expandable stent frame;
FIG. 14 schematically illustrates distally advancing an expandable stent frame of a transcatheter device into a preexisting heart valve;
FIG. 15 schematically illustrates aligning a portion of the transcatheter device with leaflets of the preexisting heart valve and deploying the severing device from a loaded state to a deployed state;
FIG. 16 schematically illustrates an exemplary method of lacerating the leaflets of the preexisting heart valve;
FIG. 17 schematically illustrates partially expanding the expandable stent frame;
FIG. 18 schematically illustrates distally advancing the transcatheter device with the partially expanded stent frame to reposition at least one of the lacerated leaflets;
FIGS. 19-20 schematically illustrate radially expanding the expandable stent frame to a fully expanded orientation;
FIG. 21 schematically illustrates proximally retracting the transcatheter device to remove the transcatheter device from the heart valve.

### DETAILED DESCRIPTION

Aspects will now be described more fully hereinafter with reference to the accompanying drawings in which example aspects are shown. Whenever possible, the same reference numerals are used throughout the drawings to refer to the same or like parts. However, this disclosure may be embodied in many different forms and should not be construed as limited to the aspects set forth herein.

As used herein, the term "about" means that amounts, sizes, formulations, parameters, and other quantities and characteristics are not, and need not be, exact, but may be approximate and/or larger or smaller, as desired, reflecting tolerances, conversion factors, rounding off, measurement error and the like, and other factors known to those of skill in the art.

Ranges can be expressed herein as from "about" one value, and/or to "about" another value. When such a range is expressed, aspects include from the one value to the other value. Similarly, when values are expressed as approximations by use of the antecedent "about," it will be understood that the value forms another aspect. It will be further understood that the endpoints of each of the ranges are significant both in relation to the other endpoint, and independently of the other endpoint.

Directional terms as used herein - for example up, down, right, left, front, back, top, bottom, upper, lower, etc. - are made only with reference to the figures as drawn and are not intended to imply absolute orientation.

Unless otherwise expressly stated, it is in no way intended that any methods set forth herein be construed as requiring that its steps be performed in a specific order, nor that with any apparatus, specific orientations be required. Accordingly, where a method claim does not actually recite an order to be followed by its steps, or that any apparatus claim does not actually recite an order or orientation to individual components, or it is not otherwise specifically stated in the claims or description that the steps are to be limited to a specific order, or that a specific order or orientation to components of an apparatus is not recited, it is in no way intended that an order or orientation be inferred in any respect. This holds for any possible non-express basis for interpretation, including matters of logic relative to arrangement of steps, operational flow, order of components, or orientation of components; plain meaning derived from grammatical organization or punctuation, and; the number or type of aspects described in the specification.

As used herein, the singular forms "a," "an," and "the" include plural references unless the context clearly dictates otherwise. Thus, for example, reference to "a" component includes aspects having two or more such components, unless the context clearly indicates otherwise.

The word "exemplary," "example," or various forms thereof are used herein to mean serving as an example, instance, or illustration. Any aspect or design described herein as "exemplary" or as an "example" should not be construed as preferred or advantageous over other aspects or designs. Furthermore, examples are provided solely for purposes of clarity and understanding and are not meant to limit or restrict the disclosed subject matter or relevant portions of this disclosure in any manner. It can be appreciated that a myriad of additional or alternate examples of varying scope could have been presented but have been omitted for purposes of brevity.

As used herein, the terms "comprising," "including," and variations thereof shall be construed as synonymous and open-ended, unless otherwise indicated. A list of elements following the transitional phrases comprising or including is a non-exclusive list, such that elements in addition to those specifically recited in the list may also be present.

The terms "substantial," "substantially," and variations thereof as used herein are intended to represent that a described feature is equal or approximately equal to a value or description. Moreover, "substantially" is intended to denote that two values are equal or approximately equal. The term "substantially" may denote values within about 10% of each other, for example, within about 5% of each other, or within about 2% of each other.

Modifications may be made to the instant disclosure without departing from the scope or spirit of the claimed subject matter. Unless specified otherwise, "first," "second," or the like are not intended to imply a temporal aspect, a spatial aspect, an ordering, etc. Rather, such terms are merely used as identifiers, names, etc. for features, elements, items, etc. For example, a first end and a second end generally correspond to end A and end B or two different ends.

Unless otherwise indicated, the terms "distal" and "proximal" are used in the following description with respect to a position or direction relative to the treating clinician. "Distal" and "distally" are positions distant from or in a direction away from the clinician, and "proximal" and "proximally" are positions near or in a direction toward the clinician. In addition, the term "self-expanding" may be used in the following description with reference to one or more valve or stent structures of the prostheses hereof and is intended to convey that the structures are shaped or formed from a material that can be provided with a mechanical memory to return the structure from a compressed or constricted delivery configuration to an expanded deployed configuration or vice versa. Non-exhaustive exemplary self-expanding materials include stainless steel, a pseudoelastic metal such as a nickel titanium alloy or nitinol, various polymers, or a so-called super alloy, which may have a base metal of nickel, cobalt, chromium, or other metal. Mechanical memory may be imparted to a wire or stent structure by thermal treatment to achieve a spring temper in stainless steel, for example, or to set a shape memory in a susceptible metal alloy, such as nitinol. Various polymers that can be made to have shape memory characteristics may also be suitable for use in aspects hereof to include polymers such as polynorborene, trans-polyisoprene, styrene-butadiene, and polyurethane. As well poly L-D lactic copolymer, oligo caprylactone copolymer and poly cyclo-octine can be used separately or in conjunction with other shape memory polymers.

Diseases associated with heart valves (e.g., a native heart valve), such as those caused by damage or a defect, can include stenosis and valvular insufficiency or regurgitation. For example, valvular stenosis causes the valve to become narrowed and hardened which can prevent blood flow to a downstream heart chamber from occurring at the proper flow rate and may cause the heart to work harder to pump the blood through the diseased valve. Valvular insufficiency or regurgitation occurs when the valve does not close completely, allowing blood to flow backwards, thereby causing the heart to be less efficient. A diseased or damaged valve, which can be congenital, age-related, drug-induced, or in some instances, caused by infection, can result in an enlarged, thickened heart that loses elasticity and efficiency. Some symptoms of heart valve diseases can include weakness, shortness of breath, dizziness, fainting, palpitations, anemia and edema, and blood clots which can increase the likelihood of stroke or pulmonary embolism. Symptoms can often be severe enough to be debilitating and/or life threatening.

Heart valve prostheses have been developed for repair and replacement of diseased and/or damaged heart valves. Such heart valve prostheses can be percutaneously delivered and deployed at the site of the diseased heart valve through catheter-based delivery systems. Such heart valve prostheses generally include a frame or stent and a prosthetic valve mounted within the frame. Such heart valve prostheses are delivered in a radially compressed or crimped configuration so that the heart valve prosthesis can be advanced through the patient's vasculature. Once positioned at the treatment site, the heart valve prosthesis is expanded to engage tissue at the diseased heart valve region to, for instance, hold the heart valve prosthesis in position.

However, over time these heart valve prostheses may also fail and require another procedure to replace the damaged heart valve. Similar to replacing a native heart valve, in order to replace a failed prosthetic heart valve, a transcatheter device including a replacement prosthesis can be employed.

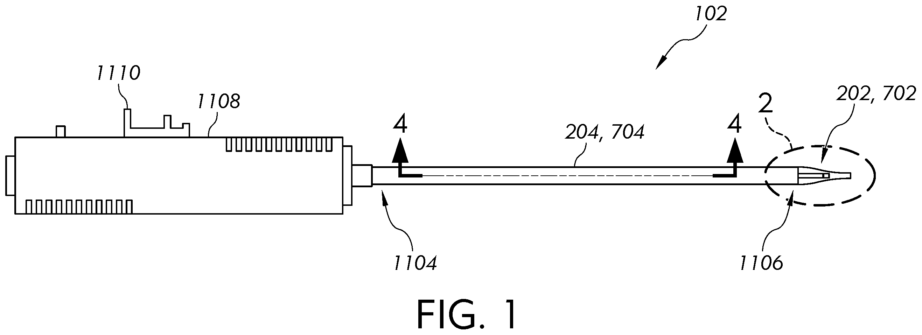

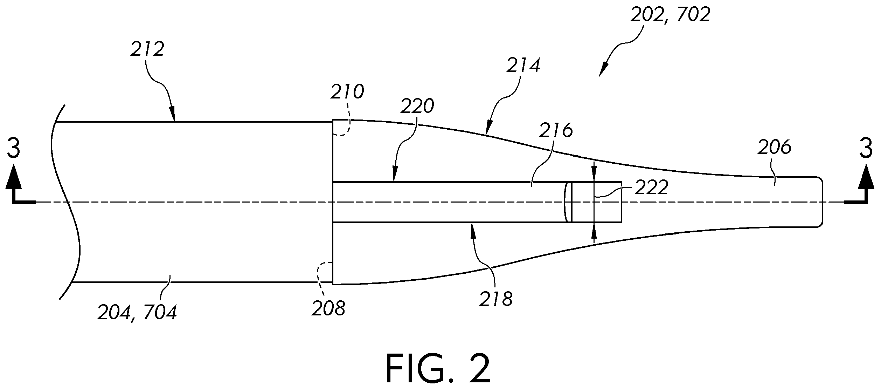

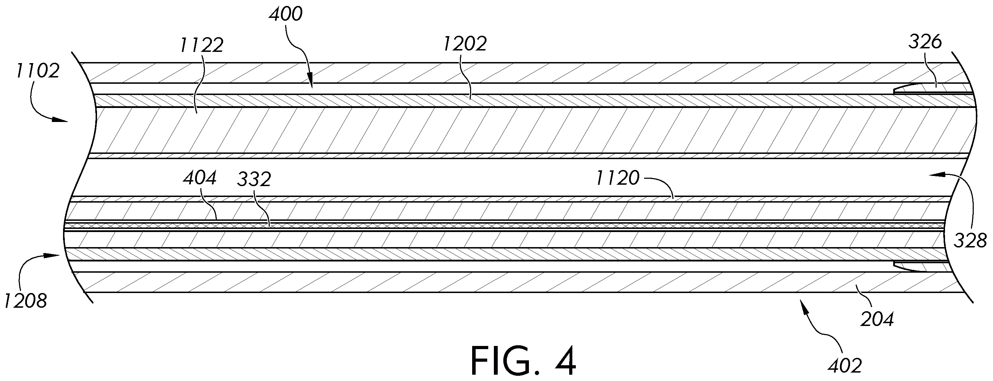

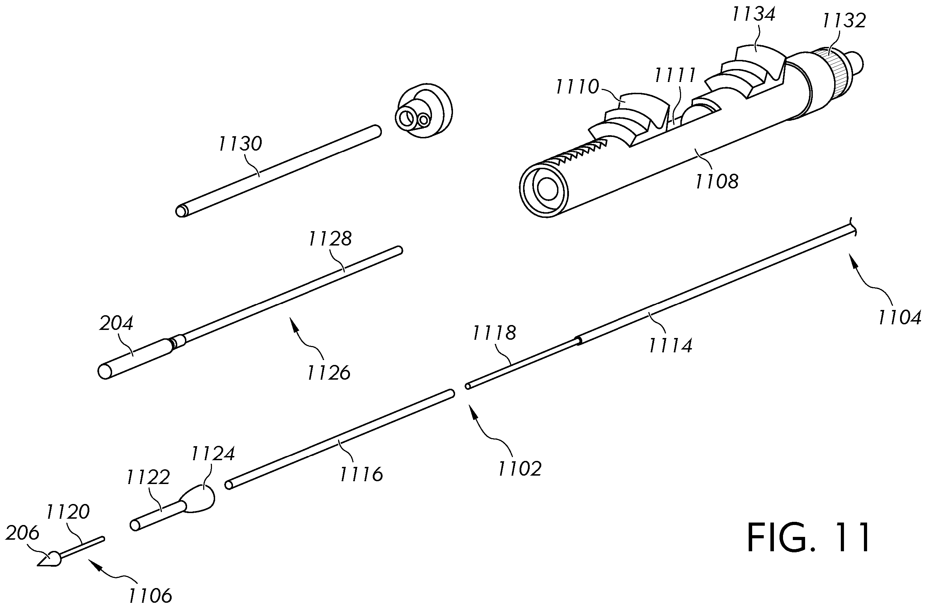

FIG. 1 illustrates an exemplary transcatheter device 102 in accordance with the present disclosure. In aspects, the transcatheter device 102 can comprise a shaft 1102 (e.g., see FIGS. 4 and 11) comprising a proximal portion 1104 and a distal portion 1106, a handle device 1108, a distal end portion 202, 702, and a sheath 204, 704. In some aspects, described in further detail hereinafter with reference to FIG. 11, the shaft 1102 can comprise one or more shafts (e.g., a shaft assembly). In aspects, as illustrated (although not visibly shown in FIG. 1 due to the sheath 204, 704, being disposed around the shaft 1102), the shaft 1102 can couple the distal end portion 202, 702 (discussed with initial reference to FIGS. 2-10) to the handle device 1108. In some examples, the handle device 1108 can be coupled to the proximal portion 1104 of the shaft 1102 and the distal end portion 202, 702 can be coupled to the distal portion 1106 of the shaft. In further aspects, the handle device 1108 can comprise a first actuator 1110, as shown.

It should be understood that the term "first actuator" is not meant to be limiting. The term "first actuator" as utilized herein should be construed to mean that an actuator can comprise more than one actuator (e.g., a first actuator, a second actuator, a third actuator, and so on) in order to actuate one or more components of the transcatheter device 102 independently from another actuator. Thus, the terms "first, second, third" and so on as utilized with referenced to actuators are merely descriptive for the purposes of distinguishing interactions between one or more actuators and one or more components of the transcatheter device 102. Accordingly, a first actuator, a second actuator, a third actuator and/or the like can actuate more than one component (e.g., a first component, a second component, etc.) of the transcatheter device 102, unless otherwise specifically denoted. It should further be understood that because the terms "first actuator, second actuator, third actuator" and/or the like denote interactions between an actuator and one or more components of the transcatheter device 102, each of the first actuator, the second actuator, the third actuator and/or the like may comprise more than one actuator. For example, the first actuator can comprise a plurality of actuators that interact with one or more components of the transcatheter device 102, while the second actuator and/or the third actuator can also comprise a plurality of actuators that interact with one or more of their respective components of the transcatheter device 102.

In aspects, the first actuator 1110 of the handle device 1108 can be used to actuate one or more components of the distal end portion 202, 702, which will become more apparent hereinafter. The transcatheter device 102 illustrated in FIG. 1 should not be construed to be limited to the above-described components. Rather, any other suitable shaft, handle device, sheath, and/or additional component may be used with the transcatheter device 102 and methods described hereinafter.

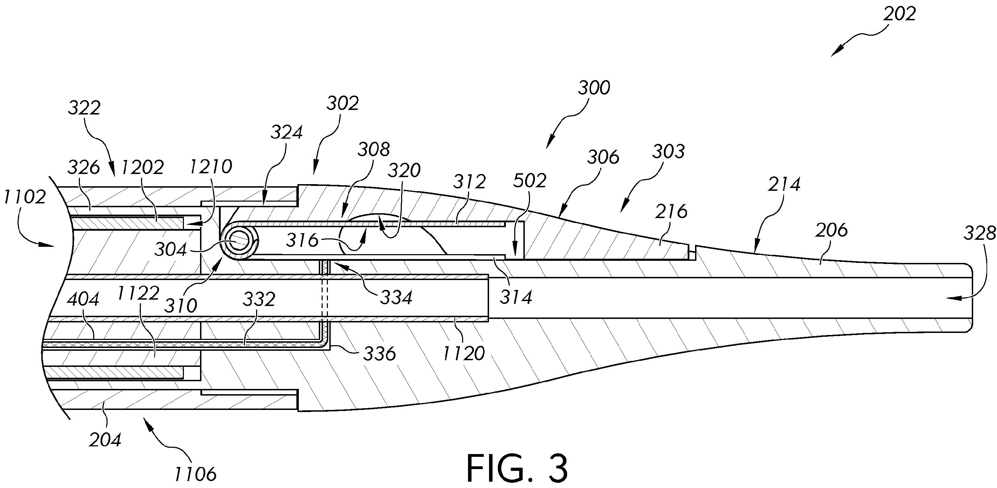

FIG. 2 illustrates the distal end portion 202, 702 of the transcatheter device 102. In aspects, the distal end portion 202, 702 can comprise a distal tip 206. In some embodiments, the distal tip 206 can be coupled to the distal portion 1106 of the shaft 1102 (see FIG. 3). For example, the distal portion 1106 of the shaft 1102 can be press fit within a lumen of the distal tip 206. Any other suitable means for coupling the distal portion 1106 of the shaft 1102 to the distal tip 206 may be utilized, such as for example, mechanical coupling (e.g., a threaded shaft that is received within a threaded hole).

In further aspects, the distal tip 206 can be any suitable shape and/or size, such as for example, a rounded conical shape to be atraumatic, thereby minimizing/preventing damage while maneuvering the transcatheter device 102 through a patient's vasculature. The distal tip 206 may further comprise any suitable material or combination of materials known to one of ordinary skill in the art of transcatheter devices, such as for example, a flexible and/or stiff polymer. In some aspects, a proximal end 208 of the distal tip 206 can be sized to abut a distal end 210 of the sheath 204, 704. For example, the proximal end 208 of the distal tip 206 can abut the distal end 210 of the sheath 204, 704 such that an atraumatic surface (e.g., a flush surface) between an outer radial surface 212 of the sheath 204, 704 and an outer radial surface 214 of the distal tip 206 is formed.

As shown in FIG. 2, the transcatheter device 102 can comprise a severing device 216 that can comprise a first edge surface 218 and a second edge surface 220 that extend perpendicular to the page to define a width 222 of the severing device 216. The severing device 216 will be described in further detail with reference to FIGS. 3-10. In some aspects, the severing device 216 can be configured to lacerate one or more leaflets of a heart valve (e.g., a preexisting heart valve such as a native heart valve or a prosthetic heart valve). For example, the severing device 216 may be beneficial in lacerating one or more leaflets of a the heart valve prior to replacing the heart valve (e.g., a native heart valve or a prosthetic heart valve).

In some non-limiting examples, the one or more leaflets can be lacerated to bisect (e.g., lacerated to divide the one or more leaflets of the heart valve into two substantially equal parts) one or more leaflets. For example, bisecting the one or more leaflets can comprise lacerating (e.g., bifurcating) the one or more leaflets to create a first lacerated portion and a second laceration portion that are substantially equal in size. In other aspects, various other locations along one or more of the one or more leaflets can be lacerated such that the first lacerated portion and the second lacerated portion are not substantially equal. In other aspects, the one or more leaflets can be lacerated to form more than two lacerated pieces (e.g., three lacerated pieces, four lacerated pieces, and so on). In some aspects, the one or more leaflets may be lacerated by varying lengths from the free end towards the cusp.

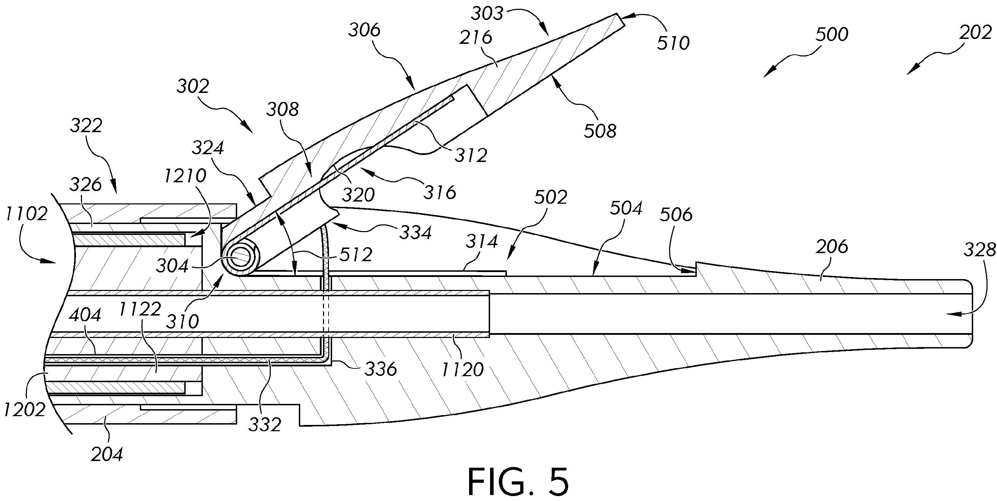

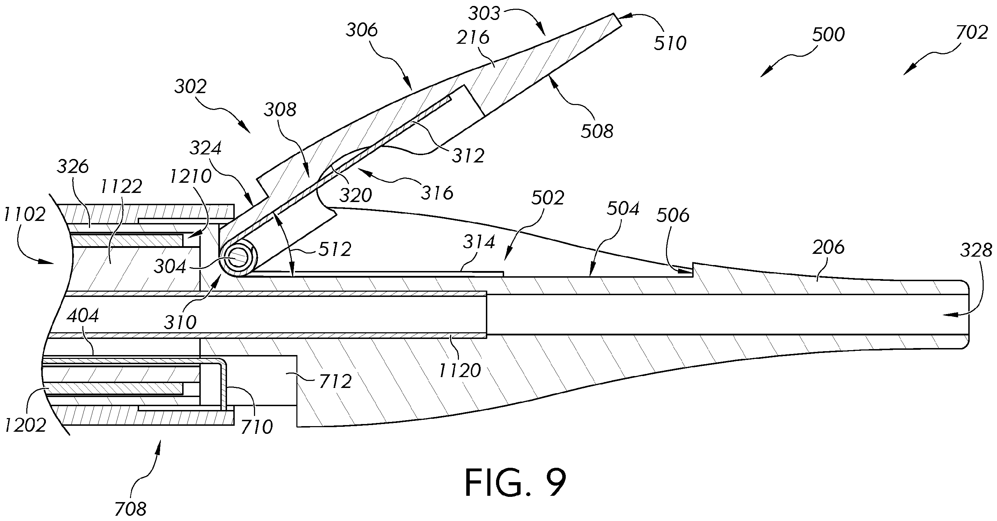

Turning to FIGS. 3-10, exemplary embodiments of the transcatheter device 102 will now be described in further detail. More specifically, FIGS. 3, 5, 7 and 9 show an exemplary cross-sectional view of the distal end portion 202, 702 of the transcatheter device 102, while FIGS. 4, 6, 8, 10 show a portion of the transcatheter device 102 located between the handle device 1108 and the distal end portion 202, 702 of FIG. 1. In aspects, as shown in FIGS. 3, 5, 7 and 9, the severing device 216 can comprise a proximal portion 302 pivotally mounted to the distal tip 206. In aspects, the severing device 216 can be pivotally mounted within a recess 502 (shown in FIGS. 5 and 9) of the distal tip 206. In some examples, the recess 502 can comprise one or more surfaces. For example, the recess 502 can comprise a first inner surface (not shown) and a second inner surface (not shown) defining a width 222 of the recess 502 that corresponds to the width 222 of the severing device 216. In some examples, the recess 502 can comprise a lower inner surface 504 (shown in FIGS. 5 and 9). In yet further examples, the recess 502 can comprise a distal end surface 506 (shown in FIGS. 5 and 9). In some examples, the distal end surface 506, as shown, can comprise a vertical planar surface extending in a direction perpendicular to the page.

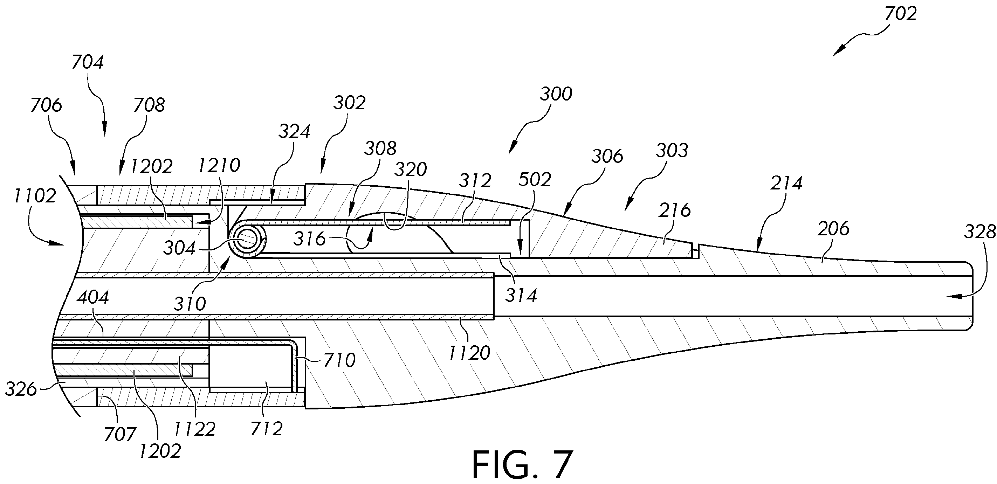

In further aspects, the severing device 216 can be configured to be pivoted from a loaded state 300 (shown in FIGS. 3 and 7) to a deployed state 500 (shown in FIGS. 5 and 9). In some aspects, the first actuator 1110 (e.g., one or more first actuators) can be configured to be activated to move the severing device 216 from the loaded state 300 to the deployed state 500. For example, a clinician (e.g., a surgeon) can interact with the first actuator 1110 (described in more detail with reference to FIG. 11) to move the severing device 216 from the loaded state 300 to the deployed state 500.

In some examples, a pin 304 can pivotally mount the proximal portion 302 of the severing device 216 to the distal tip 206. For example, the pin 304 can comprise a straight pin (e.g., a dowel pin) that is disposed within the recess 502 such that the severing device 216 can pivot between the loaded state 300 and the deployed state 500. In some such examples, the pin 304 can extend from the first inner surface (e.g., a first end of the pin 304 can be embedded within the first inner surface) of the recess 502 to the second inner surface (e.g., a second end of the pin can be embedded within the second inner surface of the recess 502) of the recess 502. Furthermore, the pin 304 (as shown) can comprise an elongated axis that extends in a direction perpendicular to the page. In this way, the severing device 216 can pivot (e.g., rotate) from the loaded state 300 to the deployed state 500 about the elongated axis of the pin 304.

In some aspects, the severing device 216 can be pivotally mounted within the recess 502 to allow for a number of suitable degrees of rotation of the severing device 216. For example, the severing device 216 can be pivotally mounted to allow for the severing device 216 to pivot from about 20 degrees to about 50 degrees between the loaded state 300 and the deployed state 500. For example, the severing device 216 can be pivotally mounted such that the severing device 216 can rotate counterclockwise about the pin 304 shown in FIGS. 3 and 7 to reach the deployed state 500 shown in FIGS. 5 and 9. The severing device 216 may rotate from about 20 degrees to about 50 degrees although greater or reduced rotation may be provided in further embodiments.

Furthermore, in some embodiments, there may be a pivot stop designed to limit the extent that the severing device 216 is able to pivot about the pin 304. For example, as shown in FIGS. 5 and 9, a pivot stop can limit the severing device 216 to pivot a predetermined angle (e.g., 45 degrees) counterclockwise about the pin 304 from the loaded state 300 shown in FIG. 3 and 7 to the deployed state 500 shown in FIGS. 5 and 9. In some aspects, the amount of degrees the severing device 216 can rotate can be selectively adjusted (e.g., by the first actuator 1110) by a clinician operating the transcatheter device 102. In some examples, the amount of degrees the severing device 216 needs to pivot may be based upon the characteristics (e.g., physiological characteristics of a native heart valve or the characteristics of a prosthetic heart valve) of the one or more leaflets (e.g., native leaflets and/or prosthetic heart valve leaflets) that need to be lacerated. For example, the severing device 216 may need to pivot a greater or lesser number of degrees depending on the size, shape, and/or angle (e.g., the angle with respect to the severing device 216) of the one or more leaflets. In other aspects, various other pins configured to allow the severing device 216 to rotate between the loaded state 300 and the deployed state 500 can be utilized (e.g., a clevis pin). The pin 304 can be a variety of suitable materials, such as for example, metal, plastic, ceramic, or any other suitable material.

As shown in FIGS. 3 and 7, in some examples, the recess 502 can be configured to receive the severing device 216 in the loaded state 300. In further examples, an outer radial surface 306 of the severing device 216 contours (e.g., radially contours) to the outer radial surface 214 of the distal tip 206 to define a flush surface when the severing device 216 is received within the recess 502 in the loaded state 300. In this way, an atraumatic surface (e.g., a surface substantially devoid of large ridges, jagged points and/or large discontinuities) can be formed. This will be beneficial in preventing and/or minimizing damage to a patient's vasculature while maneuvering the transcatheter device 102 through the vasculature of the patient.

In some aspects, the severing device 216 can comprise a lower surface 508 and a distal end surface 510. As shown, in some examples, the distal end surface 510 can comprise a vertical planar surface extending in a direction perpendicular to the page. In some examples, when the severing device 216 is in the loaded state 300, the lower surface 508 of the severing device 216 can be configured to contact a portion of the lower inner surface 504 of the recess 502. In some examples, the lower surface 508 of the severing device 216 can contact the portion of the lower inner surface 504 of the recess 502 along a plane that runs parallel to and along the lower inner surface 504 of the recess 502 and extends perpendicular to the page.

Furthermore, in some examples, when the severing device 216 is in the loaded state 300, a portion of the distal end surface 510 of the severing device 216 can contact a portion of the distal end surface 506 of the recess 502. In some examples, the distal end surface 510 of the severing device 216 can contact the portion of the distal end surface 506 of the recess 502 along a plane that runs parallel to and along the distal end surface 506 of the recess 502 and extending perpendicular to the page. In some embodiments, as shown, the distal end surface 510 of the severing device 216 does not contact the portion of the distal end surface 506 of the recess 502. In some such embodiments, as shown, a gap can be formed between the distal end surface 510 of the severing device 216 and the distal end surface 506 of the recess 502.

In some aspects, the combination of surfaces described above can define a seated interface between the severing device 216 and the recess 502 when the severing device 216 is in the loaded state 300. In this way, the flush surface, as described above, can be defined. In some embodiments, while some of the surfaces (e.g., the distal end surface 510 of the severing device 216 and the distal end surface 506 of the recess 502) have been defined above as planar surfaces, this is not meant to be limiting. For example, the surfaces do not have be planar surfaces, the surfaces can comprise any other suitable surface, such as for example, a rounded surface, a surface containing one or more discontinuities, and/or the like. While the illustrated seated interface comprises an interface between two planar surfaces, in further embodiments, the seated interface may comprise other configurations such as a tooth and gear interface, a convex/concave interface, or a ball and socket interface and/or the like.

In some embodiments, the severing device 216 can further comprise a spring 308. In some examples, as shown in FIGS. 3, 5, 7 and 9, the spring 308 can be disposed within one or more portions of the severing device 216. In some aspects, as shown, the spring 308 can comprise a torsional spring. For example, the spring 308 can comprise a coiled portion 310 comprising one or more active coils (e.g., a coil that exerts energy when the spring 308 is compressed, extended, and/or torqued), a first spring extension 312 extending from a first free end of the one or more active coils, and a second spring extension 314 extending from a second free end of one or more of the active coils. In some non-limiting examples, the coiled portion 310 can be disposed within the proximal portion 302 of the severing device 216, while the first spring extension 312 can extend in a distal direction from the first free end of the one or more active coils through one or more portions of the severing device 216. In some such examples, the second spring extension 314 can extend in a distal direction from the second free end of the one or more active coils along a portion (e.g., along the lower inner surface 504) of the recess 502. The second spring extension 314 can be coupled within the recess 502 to be fixed within the distal tip 206. For example, the second spring extension 314 can be embedded within the lower inner surface 504 of the recess 502. Alternatively, the second spring extension 314 can be coupled to the lower inner surface 504 any other suitable way, such as for example, with a bracket and/or a fixture.

In some examples, the second spring extension 314 can comprise one or more (e.g., two, three, four, etc.) protrusions (not shown) extending perpendicular to the second spring extension 314 and positioned along the length of the second spring extension 314. In this way, each protrusion of the one or more protrusions can be embedded within the first inner surface and/or the second inner surface of the recess 502 to fix the second spring extension 314 therein. It should be understood that the above-described (and illustrated) spring 308 is not meant to be limiting and any other suitable spring may be utilized, such as for example, other torsional springs comprising various shapes and sizes, or a compression spring. For example, a compression spring can be disposed within the lower inner surface 504. Leaf springs or other spring configurations can be used in further examples.

In some aspects, the spring 308 can be configured to bias the severing device 216 to the deployed state 500. For example, in the orientation illustrated in the drawings, the spring 308 can be configured to exert a counterclockwise rotational force on the severing device 216 such that the severing device 216 is biased to rotate towards the deployed state 500 shown in FIGS. 5 and 7. The spring 308 can provide the counterclockwise rotational torsional biasing force by potential energy stored in the above-described coils. In some examples, when the first spring extension 312 and the second spring extension 314 are in their natural, untensioned state (e.g., when the second spring extension 314 is not under a compressive load), the first spring extension 312 and the second spring extension 314 will reside at a preset angle 512 from one another. In some examples, the preset angle 512 may serve as a natural pivot stop, such as for example, the pivot stop described previously. Furthermore, by moving the first spring extension 312 away from its natural, untensioned state (e.g., by applying a compressive load to the first spring extension 312) towards a tensioned state (e.g., by moving the severing device 216 to the loaded state 300 as shown in FIGS. 3 and 7), the first spring extension 312 will be configured to bias the severing device 216 to the deployed state 500. In further examples, the spring may be wound such that the spring applies a biasing force to the severing device 216 in both the loaded state and the deployed state. In such examples, an interaction between the distal tip and the severing device may act as a stop to limit the degree that the severing device pivots relative to the distal tip.

In further aspects, the spring 308 can comprise a region 316 that is configured to be energized in the deployed state 500. For example, the region 316 can be configured to receive an electrical current, such as for example, from a power source (e.g., battery, generator, electrical outlet, etc.). The region 316 that is energized can be beneficial to sever the one or more leaflets as well as cauterize one or more leaflets of a heart valve (e.g., a native heart valve and/or a prosthetic heart valve). For example, the region 316 can be configured to cauterize one or more leaflets, such as for example, to bisect the one or more leaflets, as described above. In some aspects, as illustrated, the region 316 of the severing device 216 can comprise an exposed portion. Thus, the region 316 of the spring 308 can be exposed to the surrounding area (e.g., the vasculature of a patient, a lumen within a heart valve) such that the region 316 of the spring 308 may contact one or more leaflets of a heart valve in order to sever (e.g., bifurcate) and/or cauterize the one or more leaflets. In some aspects, some portions of the spring 308 may be insulated. In this way, the insulated portions will not be active when the severing device 216 is in the deployed state 500 to avoid damaging certain areas near the target site. Although not shown, in further embodiments, the severing device may comprise a blade designed to lacerate the leaflet with or without being energized. For example, a scalpel can be provided that is not designed to be energized that can be distally advanced to sever the leaflet. In further embodiments, a the region 316 can be sharpened into a blade to facilitate lacerating of the leaflet while simultaneously cauterizing the leaflet.

In aspects, the exposed portion can extend perpendicular to the page from the first edge surface 218 to the second edge surface 220 (see FIG. 2). In some aspects, the exposed portion can be defined by one or more recesses 320 that extend into the lower surface 508 of the severing device 216 towards the outer radial surface 306 of the severing device 216. The one or more recesses 320 can extend any number of suitable distance from the lower surface 508 of the severing device 216 towards the outer radial surface 306 of the severing device 216. In some non-limiting examples, the one or more recesses 320 can extend about half the distance between the lower surface 508 of the severing device 216 and the outer radial surface 306 of the severing device 216. In other examples, the recesses 320 can be greater than or less than half the distance between the lower surface 508 of the severing device 216 and the outer radial surface 306 of the severing device 216.

In aspects, the sheath 204, 704 can be disposed around the shaft 1102. In some embodiments, the sheath 204, 704 can be configured to translate relative to the distal tip 206. The term "translate" as used herein, can mean that the sheath 204, 704 can move, slide, shift, and/or the like. In some examples, the sheath 204, 704 can be configured to proximally and/or distally translate relative to the distal tip 206. In further examples, the sheath 204, 704 can also be configured to rotate (e.g., 360 degrees) relative to the distal tip 206 about an elongated axis of the transcatheter device 102.

In some aspects, the sheath 204, 704 can comprise a proximal portion 402, 706 (see FIGS. 4 and 7) and a distal portion 322, 708 (see FIGS. 3 and 7). In aspects, the loaded state 300 can comprise the distal portion 322, 708 of the sheath 204, 704 disposed around the proximal portion 302 of the severing device 216. In some non-limiting examples, as illustrated, the proximal portion 302 of the severing device 216 can comprise a recessed surface 324 that is configured to be received within the distal portion 322, 708 of the sheath 204. For example, the distal portion 322, 708 of the sheath 204, 704 can be positioned over top of the recessed surface 324, as shown in FIGS. 3 and 7, when the severing device 216 is in the loaded state 300. In this way, the distal portion 322, 708 of the sheath 204, 704 can provide a force to selectively lock the severing device 216 in the loaded state 300. For example, the distal portion 322, 708 of the sheath 204, 704 can exert a force that counteracts the biasing force of the spring 308 (as previously described). In this case, when the distal portion 322, 708 of the sheath 204, 704 is positioned over top of the proximal portion 302 of the severing device 216, the force will act in a radial direction towards the shaft 1102 of the transcatheter device 102. Meanwhile, the counterclockwise rotational force (e.g., the biasing force) of the spring 308 will act against (e.g., in the opposite direction) the force of the distal portion 322, 708 of the sheath 204, 704. Thus, in some examples, proximally translating (e.g., proximally retracting) the distal portion 322, 708 of the sheath 204, 704 relative to the distal tip 206, will release the severing device 216 to pivot under the force of the spring from the loaded state 300 to the deployed state 500. This is a result of the radial compression force being removed from the proximal portion 302 of the severing device 216, thereby allowing the spring 308 to pivot towards its natural, untensioned state.

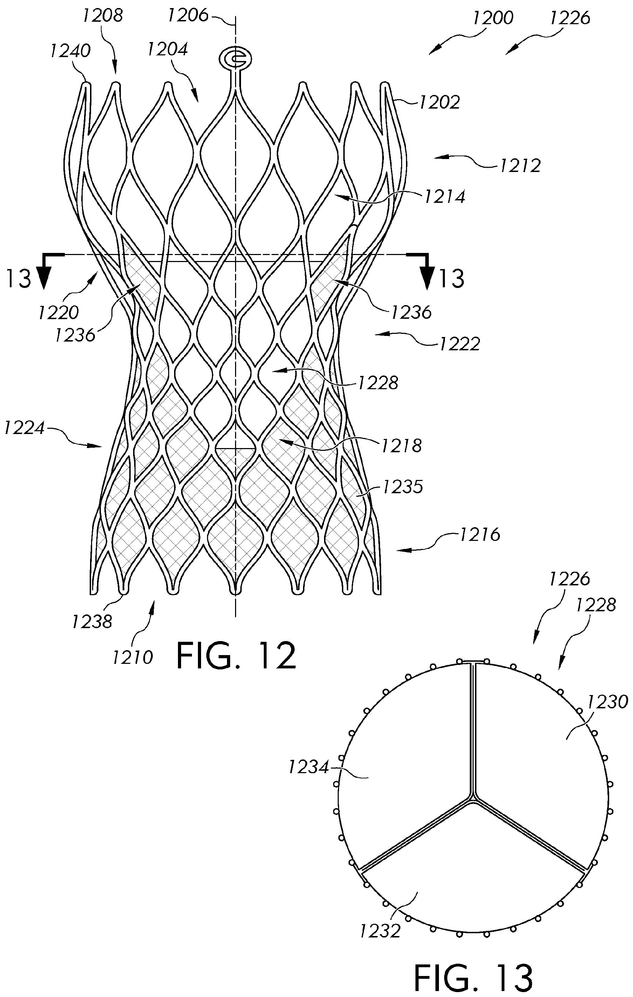

With additional reference to FIGS. 12 and 13, in some aspects, the transcatheter device 102 can further comprise an expandable stent frame 1202. For example, the expandable stent frame 1202 may be a self-expandable stent frame, such as for example, a nitinol self-expanding stent frame. In other non-limiting examples, the expandable stent frame 1202 may include an expansion device (e.g., a balloon) configured to expand the expandable stent frame 1202. In aspects, the expandable stent frame 1202 can comprise an inner lumen 1204 extending along an elongated axis 1206. In some examples where the expandable stent frame 1202 includes the expansion device, the expansion device can be positioned within the inner lumen 1204. For example, the expansion device can comprise a balloon configured to receive saline, a contrast solution, or compressed air that may be delivered to the balloon to expand the expandable stent frame 1202.

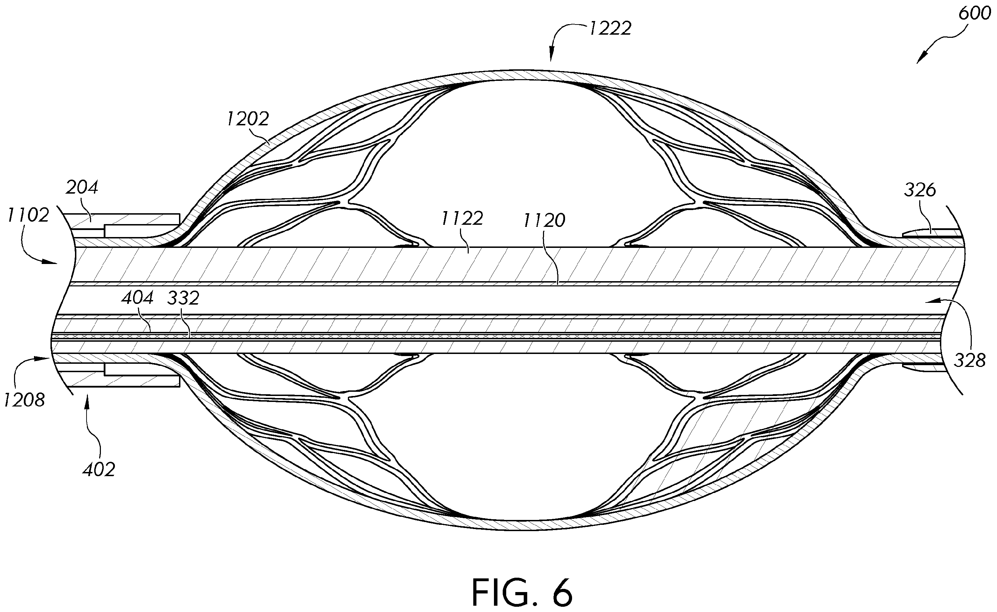

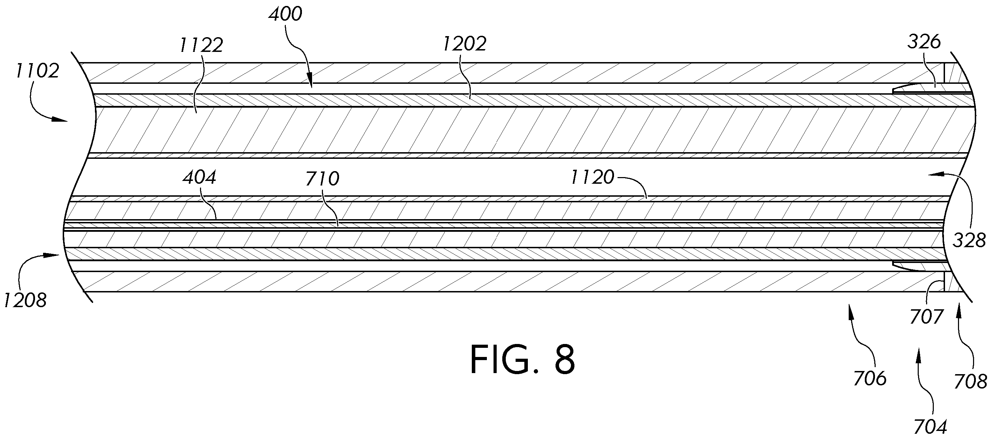

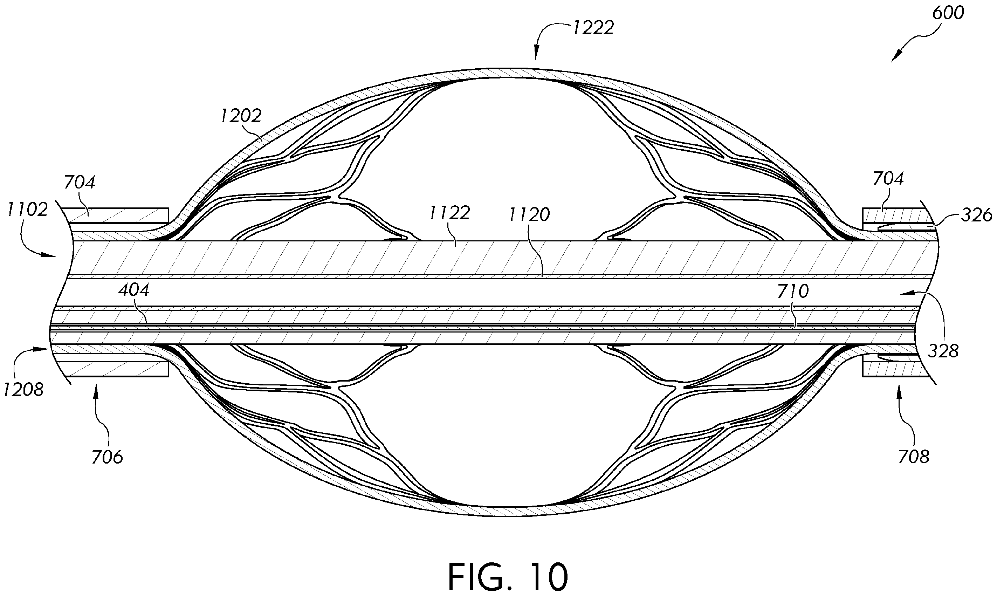

In aspects, the expandable stent frame 1202 can comprise a proximal end 1208 (outflow end) and a distal end 1210 (inflow end). The expandable stent frame 1202 can extend from the proximal end 1208 to the distal end 1210. In aspects, the proximal end 1208 can be configured to be received within the sheath 204, 704. In some non-limiting examples, the proximal end 1208 can be slidably disposed within the sheath 204, 704. In some embodiments, the expandable stent frame 1202 can be configured to achieve a contracted orientation 400 (shown in FIGS. 4 and 8), a partially expanded orientation 600 (shown in FIGS. 6 and 10), and a fully expanded orientation 1200 (shown in FIGS. 12 and 20-21).

The term "fully expanded orientation" should be construed to mean that the expandable stent frame 1202 cannot expand any further with respect to the location at which the expandable stent frame 1202 has been deployed (i.e., fully released from the transcatheter device 102). For example, when the expandable stent frame 1202 is discussed with reference to an implanted prosthetic heart valve or a native heart valve, the term "fully expanded orientation" will refer to the expandable stent frame 1202 being fully released from the transcatheter device 102 whereby the expandable stent frame 1202 has contacted the implanted prosthetic heart valve or the native heart valve such that the expandable stent frame 1202 can no longer expand. Accordingly, the term "fully expanded orientation" can mean that the expandable stent frame 1202 cannot radially expand any further with respect to the location at which it has been deployed and implanted even though the expandable stent frame 1202 is still capable of further radial expansion if the expandable stent frame 1202 were free from surrounding structure. In some cases, the term "fully expanded orientation" may refer to the expandable stent frame 1202 not being capable of any further radial expansion regardless of the location at which the expandable stent frame 1202 is deployed (e.g., the expandable stent frame 1202 cannot radially expand any further, even when free from surrounding structure).

In some aspects, a proximal portion 1212 of the expandable stent frame 1202 adjacent the proximal end 1208 can comprise a first plurality of cell structures 1214. In further aspects, a distal portion 1216 of the expandable stent frame 1202 adjacent the distal end 1210 can comprise a second plurality of cell structures 1218. As show, the first plurality of cell structures 1214 and the second plurality of cell structures 1218 can comprise diamond-shaped cell structures.

It should be understood that the expandable stent frame 1202 is merely exemplary and any other suitable expandable stent frames may be utilized with the exemplary transcatheter device 102 described herein. Furthermore, the first plurality of cell structures 1214 and the second plurality of cell structures 1218 should not be limited to diamond-shaped cell structures and should not be limited to the number of cell structures and/or size of the cell structures shown in FIG. 12. Accordingly, more or less suitable cell structures with a variety of different shapes and sizes can be employed with the transcatheter device 102.

In aspects, the proximal portion 1212 can comprise a first outwardly bulging circumferential portion 1220. The first outwardly bulging circumferential portion 1220 can encircle the inner lumen 1204 of the expandable stent frame 1202 and may bulge radially outward with respect to at least an intermediate portion 1222 of the expandable stent frame 1202. In some aspects, the first outwardly bulging circumferential portion 1220 can taper towards the distal portion 1216 and adjoin a first end portion of the intermediate portion 1222. In aspects, a second end portion of the intermediate portion 1222 can adjoin the distal portion 1216. The distal portion 1216 can comprise a second outwardly bulging circumferential portion 1224 that can encircle the inner lumen 1204 and may bulge radially outward with respect to at least the intermediate portion 1222 of the expandable stent frame 1202. In some aspects, the second outwardly bulging circumferential portion 1224 can taper towards the proximal portion 1212 of the expandable stent frame 1202. In aspects, as shown, the second outwardly bulging circumferential portion 1224 can have a smaller diameter than the first outwardly bulging circumferential portion 1220. In other aspects, the second outwardly bulging circumferential portion 1224 can have a greater diameter than the first outwardly bulging circumferential portion 1220. In yet another aspect, the second outwardly bulging circumferential portion 1224 can have a substantially equal diameter to that of the first outwardly bulging circumferential portion 1220.

In some aspects, the transcatheter device 102 can comprise a skirt 1235 coupled to the expandable stent frame 1202. As shown, in some examples, the skirt 1235 can be coupled to the distal portion 1216 of the expandable stent frame 1202 and can be coupled to portions of the intermediate portion 1222. For example, the skirt can be coupled to the intermediate portion 1222 of the expandable stent frame 1202 and extend from the intermediate portion 1222 towards the distal end 1210 (e.g., the skirt can extend to any portion of the distal portion 1216 or to the distal end 1210). In other examples, the skirt can extend along any length of the expandable stent frame 1202. For example, the skirt can extend along any length of the expandable stent frame 1202 defined between the proximal end 1208 and the distal end 1210 of the expandable stent frame 1202. The skirt 1235 can be coupled to the expandable stent frame 1202 by any suitable coupling means. For example, skirt can be coupled to the expandable stent frame 1202 by stitching, such as for example, coupling the skirt to the expandable stent frame 1202 by a stitching pattern that extends from the intermediate portion 1222 of the expandable stent frame 1202, and along the distal portion 1216 of the expandable stent frame 1202 (i.e., along one or more of the second plurality of cell structures 1218).

In some aspects, the skirt can comprise a radiopaque polymer. For example, a radiopaque polymer may be beneficial in facilitating a clinician in determining when the expandable stent frame 1202 has been properly positioned within a patient. Any suitable polymer known in the art may be utilized. In other aspects, the skirt can comprise a fabric. For example, the fabric may comprise any suitable material such as, but not limited to, woven polyester such as polyethylene terephthalate, polytetrafluoroethylene (PTFE), or other biocompatible materials.

In some aspects, the transcatheter device 102 can comprise a prosthetic heart valve 1226 comprising the expandable stent frame 1202. For example, as best shown in FIG. 13, the prosthetic heart valve 1226 can comprise one or more leaflets 1228 (e.g., two or more leaflets). In some examples, as shown, the one or more leaflets 1228 can comprise three leaflets to emulate a tricuspid valve. For example, the one or more leaflets 1228 can comprise a first leaflet 1230, a second leaflet 1232, and a third leaflet 1234. In some aspects, the one or more leaflets 1228 can comprise two leaflets, such as for example, to emulate a bicuspid valve. Any other suitable number of leaflets may be utilized.

In some aspects, the expandable stent frame 1202 can comprise one or more commissure points 1236. The commissure points 1236 can be utilized to couple the valve structure (e.g., the one or more leaflets 1228) of the prosthetic heart valve 1226 to the expandable stent frame 1202. In some aspects, the valve structure can be coupled to the expandable stent frame 1202 by a stitching pattern, such as for example, by stitching the valve structure to the expandable stent frame 1202 at the one or more commissure points 1236, such as shown in FIG. 12. Any other suitable means of coupling the valve structure to the expandable stent frame 1202 may be utilized.

In further aspects, the one or more commissure points 1236 can comprise one or more radiopaque markers (e.g., one radiopaque marker, two radiopaque markers, three radiopaque markers, etc.). For example, one or more radiopaque markers can be located on any one of the one or more commissure points 1236, such as for example, having one or more radiopaque markers on each of the one or more commissure points 1236. The one or more radiopaque markers can be the same or similar as the radiopaque markers described in further detail hereinafter. In this way the expandable stent frame and/or the valve structure can be located by a clinician (e.g., a surgeon) when maneuvering the expandable stent frame 1202 through the vasculature and/or within a heart valve of a patient.

In aspects, as illustrated, the distal end 1210 of the expandable stent frame 1202 can comprise a plurality of crowns 1238. In further aspects, at least one crown of the plurality of crowns 1238 can comprise a paddle (not shown) configured to be received by the distal tip 206. In one example, "received by" can mean "received within." For example, the paddle can be received within the distal tip 206. In some aspects, each crown of the plurality of crowns 1238 can comprise a paddle that is configured to be received by the distal tip 206. In some examples, each crown of the plurality of crowns 1238 can comprise a paddle. For example, if the plurality of crowns 1238 comprises a first crown and a second crown then the first crown and the second crown can comprise a paddle. In some examples, if the plurality of crowns 1238 comprises a first crown, a second crown, and a third crown then the first crown, the second crown, and the third crown can each comprise a paddle. Any other suitable combination for the number of crowns 1238 and paddles may be acceptable (e.g., one crown with one paddle, two crowns with one paddle, three crowns with two paddles, and so on). In some aspects, the paddle can comprise a solid paddle (e.g., no holes). In alternative aspects, the paddle can comprise a hollow paddle. For example the paddle can comprise a wire structure that comprises a cut out portion within the center of the paddle.

In some aspects, not shown, the plurality of crowns 1238 can comprise three or less crowns 1238. In an example with three crowns, each crown (e.g., a first crown, a second crown, and a third crown) may be equidistantly spaced around the distal end 1210 of the expandable stent frame 1202. In some examples, the three crowns can be skewed around the distal end 1210 of the expandable stent frame 1202 (e.g., not equidistantly spaced). It should be noted that the expandable stent frame 1202 is not limited to three crowns. For example, the plurality of crowns 1238 can comprise two crowns. In some such examples, the plurality of crowns can comprise a first crown and a second crown. In such an example, the first crown and the second crown can be diametrically opposed (e.g., a circumferential distance from one crown to another is equal when measured clockwise and/or counterclockwise) around the distal end 1210 of the expandable stent frame 1202. In other examples with two crowns, the two crowns do not have to be diametrically opposed, and may have any other suitable position around the distal end 1210 of the expandable stent frame 1202.

In some aspects, the distal end 1210 can comprise more than three crowns 1238 (e.g., four crowns, five crowns, and so on) or less than two crowns (e.g., one crown). However, reducing the number of crowns may be beneficial in facilitating the loading of the expandable stent frame 1202 within the transcatheter device 102 by reducing the amount of crowns that will need to be received by (e.g., within) the distal tip 206.

In aspects, the proximal end 1208 of the expandable stent frame 1202 can comprise a plurality of crowns 1240. For example, the proximal end 1208 of the expandable stent frame 1202 can comprise one crown, two crowns, three crowns, and so on. In some aspects, at least one crown of the plurality of crowns 1240 of the proximal end 1208 can comprise a paddle (not shown) configured to be received by the sheath 204, 704. In one example, "received by" can mean "received within." For example, the paddle can be received within the sheath 204, 704. In some aspects, any other number of paddles can be provided. For example, each crown of the plurality of crowns 1240 can comprise a paddle. The plurality of crowns 1240 can be positioned around the proximal end 1208 of the expandable stent frame 1202 similarly as described above with reference to the plurality of crowns 1238 of the distal end 1210 of the expandable stent frame 1202.

FIGS. 3-5, and 7-9 illustrate the expandable stent frame 1202 in the contracted orientation 400. In aspects, the contracted orientation 400 can comprise the proximal end 1208 of the expandable stent frame 1202 being in a collapsed state within the sheath 204, 704. For example, the sheath 204, 704 can radially compress (e.g., apply a radial compression force to the proximal end 1208) the proximal end 1208 of the expandable stent frame 1202 to compress the proximal end 1208 into the collapsed state. In further aspects, the contracted orientation 400 can further comprise the distal end 1210 of the expandable stent frame 1202 being in the collapsed state within the distal tip 206. For example, the distal tip 206 can comprise a retaining element 326 that retains the distal end 1210 of the expandable stent frame 1202 therein. In some examples, the retaining element 326 can comprise one or more lumens for retaining the distal end 1210 of the expandable stent frame 1202. In some examples, the one or more lumens can comprises one or more slots for receiving the paddles (described above) within the distal tip 206. In some aspects, the contracted orientation 400 can comprise the expandable stent frame 1202 being in the collapsed state around the shaft 1102. In further aspects, the shaft 1102 can comprise an elongated lumen 328 configured to receive an object. For example, the elongated lumen 328 can be configured to receive a guidewire, such as for example, for the transcatheter device 102 to be guided along while being maneuvered through the vasculature of a patient.

FIGS. 6 and 10 illustrates the transcatheter device 102 in the partially expanded orientation 600. In aspects, the sheath 204, 704 is configured to be proximally and/or distally retracted relative to the expandable stent frame 1202. For example, as shown, the sheath 204, 704 has been proximally retracted. In some aspects, proximally retracting the sheath 204, 704 expands the expandable stent frame 1202 from the contracted orientation 400 to the partially expanded orientation 600. In aspects, the intermediate portion 1222 of the expandable stent frame 1202 defined between the proximal end 1208 (as shown generally) and the distal end 1210 (see FIGS. 3 and 7 showing the distal end 1210) is configured to bend radially outward in the partially expanded orientation 600. For example, the partially expanded orientation 600 can comprise the intermediate portion 1222 being bent radially outward relative to the sheath 204, 704. In some examples, "bend" means that the intermediate portion 1222 bows radially outwardly to form a substantially convex outer profile, as shown. In some examples, the convex outer profile can resemble a balloon-shaped structure.

In some aspects, the partially expanded orientation 600 can further comprise the proximal end 1208 of the expandable stent frame 1202 being in the collapsed state within the sheath 204, 704. For example, the sheath 204, 704 can apply the previously described radial compression force to maintain the proximal end 1208 of the expandable stent frame 1202 in the collapsed state. In aspects, the partially expanded orientation 600 can further comprise the distal end 1210 of the expandable stent frame 1202 being in the collapsed state within the distal tip 206. For example, the distal tip 206 can comprise the retaining element 326 that retains the distal end 1210 of the expandable stent frame 1202 (such as that described above for the contracted orientation 400). In aspects, the proximal end 1208 and the distal end 1210 of the expandable stent frame 1202 may be held in their respective portions of the sheath by a tip/crown capture mechanism. One example of a crown capture mechanism may be one or more paddles connected to a crown of the prosthesis that is configured to be received within a pocket on a component of the transcatheter device 102, such as a spindle on the shaft 1102. The sheath may keep the paddle locked in the pocket until the sheath is translated past the pocket, at which point the paddle can release from the pocket. This is merely one type of crown capture mechanism; however, any suitable mechanism may be used (e.g., suture-based retention mechanisms).

In some examples not shown, the transcatheter device 102 may further comprise one or more releasable cords around the expandable stent frame 1202 when in the contracted orientation 400 and the partially expanded orientation 600. For example, employing a releasable cord around the intermediate portion 1222 of the expandable stent frame 1202 can maintain the expandable stent frame 1202 in the contracted orientation 400 after the sheath 204, 704 has been proximally retracted. In this way, releasing the releasable cord can bend the expandable stent frame 1202 radially outward as shown in FIGS. 6 and 10. Similarly, releasable cords can be utilized with the proximal end 1208 and the distal end 1210 of the expandable stent frame 1202 as described with reference to the intermediate portion 1222 of the expandable stent frame 1202.

Turning now to FIGS. 3-6, a specific embodiment of the distal end portion 202 and the portion of the transcatheter device 102 located between the handle device 1108 and the distal end portion 202 will now be discussed in further detail. As shown best in FIGS. 3 and 5, the first actuator 1110 can comprise a tether 332 configured to pivot the severing device 216 from the deployed state 500 to the loaded state. For example, when the severing device 216 is in the deployed state 500 (as shown in FIG. 5), tension can be applied to the tether 332 (e.g., by activating the first actuator 1110). As the tension in the tether 332 overcomes the biasing force of the spring 308, the severing device 216 will pivot from the deployed state 500 to the loaded state 300.

In some further aspects, after the severing device 216 has been pivoted from the deployed state 500 to the loaded state 300, the tension in the tether 332 can be released to pivot the severing device 216 from the loaded state 300 back to the deployed state 500. For example, a clinician may need to move the severing device 216 from the deployed state 500 to the loaded state 300 temporarily, such as for example, to readjust the transcatheter device 102 and/or change to a different leaflet to be lacerated. In some such examples, the clinician can then move the severing device 216 back to the deployed state 500 and continue utilizing the lacerating device as previously described to lacerate more leaflets. It should be understood this is merely one exemplary reason to move the severing device 216 from the deployed state 500 to the loaded state 300 and then back to the deployed state 500, there may be many other reasons a clinician may need to move the severing device 216 as described and is thus not meant to be limiting. It should be understood that the severing device 216 can be moved (e.g., pivoted) between the loaded state 300 and the deployed state 500 as many times as required, as described herein.

In some embodiments, the tether 332 can be utilized additionally and/or alternatively to the sheath 204. For example, in some aspects, the distal portion 322 of the sheath 204 can be disposed around the proximal portion 302 of the severing device 216 as described above, but proximally retracting the sheath 204 does not have to move the severing device 216 from the loaded state 300 to the deployed state 500 if there is tension in the tether 332. In some such examples, this may be beneficial in preventing the severing device 216 from accidently deploying. In other such examples, this may be beneficial to be able to expand the expandable stent frame 1202 from the contracted orientation 400 to the partially expanded orientation 600 without moving the severing device 216 from the loaded state 300 to the deployed state 500. In other examples, the distal portion 322 of the sheath 204 does not need to be disposed around the proximal portion 302 of the severing device 216 at all. In this case, the tether 332 can maintain the severing device 216 in the loaded state 300 (e.g., by maintaining the tension in the tether 332) and move the severing device 216 from the loaded state 300 to the deployed state 500 (e.g., by releasing the tension in the tether 332). It should be understood that the tether 332 may also be omitted in some embodiments, and the distal portion 322 of the sheath 204 can operate as described above to move the severing device 216 between the loaded state 300 and the deployed state 500. (e.g., by proximally and/or distally advancing the sheath 204).

In some aspects, the tether 332 can be coupled to the proximal portion 302 of the severing device 216. In some examples, the tether 332 can be coupled to a lower portion 334 of the proximal portion 302 of the severing device 216. For example, the tether 332 can be coupled along any length of the lower surface 508 of the proximal portion 302 of the severing device 216. In some embodiments, the tether 332 can be coupled to the lower portion 334 to be centrally positioned between the width 222 of the severing device 216. The term centrally positioned as used herein should be construed to mean that tether 332 is within a central third of the width 222. In this way, the width 222 can be viewed, for the purposes of clarity, as being divided into three equal sections (e.g., each section being one third of a total length of the width 222), wherein a top section (e.g., while viewing FIG. 2) can be one third of the total length of the width 222, a bottom section (e.g., while viewing FIG. 2) can be one third of the total length of the width 222, and a central section (e.g., one third of the total length of the width 222) can be between the top section and the bottom section. Accordingly, the tether 332 can be positioned at any location within the central section located between the top section and the bottom section. The location at which the tether 332 is coupled to the severing device 216 as described above is not meant to be limiting. Any other suitable location along the severing device 216 may be utilized, such as for example, coupling the tether 332 to a distal portion 303 of the severing device 216 (not shown). The tether 332 may be coupled to the severing device 216 by any suitable coupling means known in the art, such as for example, a loop and hook connection, a double loop couple and/or the like.

In some aspects, as shown in FIG. 4 and 6, the tether 332 can be disposed within the shaft 1102, such as for example, to route the tether 332 from the first actuator 1110 to the distal tip 206. In some aspects, the tether 332 can be disposed within the elongated lumen 328 and configured to translate therein (e.g., proximally and/or distally translate therein). In some aspects, the shaft 1102 can comprise an inner shaft 1120 and an outer shaft 1122. In some such aspects, the outer shaft 1122 can comprise one or more passageways 404 that extend in a longitudinal direction of the transcatheter device 102. The one or more passageways 404 can be configured to receive the tether 332 therein (e.g., slidably receive the tether 332 therein). In some aspects, the tether 332 can be disposed between the inner shaft 1120 and the outer shaft 1122. Such as for example, by leaving a gap between at least a portion of the inner shaft 1120 and the outer shaft 1122 to route the tether 332 therethrough. In other examples not shown, the inner shaft 1120 can comprise one or more passageways 404 that extend in a longitudinal direction of the transcatheter device 102. The one or more passageways 404 can be configured to receive the tether 332 therein (e.g., slidably receive the tether 332 therein). In yet another aspect, the shaft 1102 can comprise only a single shaft (e.g., such as only including the inner shaft 1120). In some such aspects, the single shaft can comprise one or more passageways 404 that extend in a longitudinal direction of the transcatheter device 102. The one or more passageways 404 can be configured to receive the tether 332 therein (e.g., slidably receive the tether 332 therein).

In some aspects, the one or more passageways 404 can extend into a portion of the distal tip 206 (e.g., a proximal portion of the distal tip 206). In some such examples, the distal tip 206 can comprise one or more passageways 336 for receiving the tether therein (e.g., slidably receiving the tether 332 therein). For example, the one or more passageways of the distal tip 206 can align with the one or more passageways 404 of the shaft 1102 to receive the tether 332 from the distal portion 1106 of the shaft 1102. The tether can then be routed within the one or more passageways 336 of the distal tip 206 to be coupled to the severing device 216.

In some aspects, the tether 332 can be a variety of suitable materials. For example, the tether 332 can be a resilient material with a tendency to conform back to its original shape. For example, materials with a relatively small Young's modulus can be suitable, such as for example, elastomers. In some aspects, the tether 332 can be a material with a relatively high Young's modulus, such as for example, alloys and/or a variety of other metals. In some aspects, the tether 332 can be formed from high-strength synthetic fibers, such as for example, nylon and/or polyester. The tether 332 material should by no means be limited to the above-mentioned materials and can be any other suitable material to move the severing device 216 between the loaded state 300 and the deployed state 500.

It should be understood that while the tether 332 has been described as being routed through/within the shaft 1102 and through/within the distal tip 206, the tether 332 should not be limited to these embodiments. Any other suitable means of connecting the tether 332 to the severing device 216 and the first actuator 1110 may be utilized, such as for example, by routing the tether 332 from the severing device 216 to the outer radial surface 214 of the distal tip 206 and along the sheath 204 to be coupled to the first actuator 1110.

Turning now to FIGS. 7-10, a specific embodiment of the distal end portion 702 and the portion of the transcatheter device 102 located between the handle device 1108 and the distal end portion 702 will now be discussed in further detail. As shown best in FIGS. 7 and 9, the proximal portion 706 and the distal portion 708 of the sheath 704 can be configured to translate and/or rotate relative to one another. In some embodiments, the sheath 704 can comprise a circumferential split 707 that separates the proximal portion 706 and the distal portion 708. In some aspects, the circumferential split 707 can be configured to allow at least one of the proximal portion 706 or the distal portion 708 to rotate, e.g., 360 degrees, relative to the other of the proximal portion 706 or distal portion 708. In addition or alternatively, the circumferential split 707 can be configured to permit at least one of the proximal portion 706 or the distal portion 708 to translate relative to the other of the proximal portion 706 or the distal portion 708, such as for example, by relatively sliding or shifting. As shown, the proximal portion 706 and the distal portion 708 can abut one another at the circumferential split 707 although they can overlap one another in further embodiments.

In some aspects, the first actuator 1110 can comprise a stiff member 710 (e.g., a shaft, wire and/or the like) that is coupled to the distal portion 708 of the sheath 704 that can be configured to translate (e.g., proximally and/or distally) the distal portion 708 of the sheath 704. For example, the stiff member 710 can be coupled to the first actuator 1110 to translate the distal portion 708 of the sheath 704, while the proximal portion 706 of the sheath 704 is coupled to a different actuator (e.g., a second independent actuator). In some aspects, the proximal portion 706 is coupled to the first actuator 1110 to translate the proximal portion 706, while the distal portion 708 is coupled to a different actuator (e.g., a second independent actuator). In other aspects, the proximal portion 706 and the distal portion 708 can both be coupled to the first actuator 1110 where the first actuator 1110 comprises a plurality of actuators that can each be independently actuated. In some aspects, proximally retracting the distal portion 708 of sheath 704 (as described generally above) by way of the stiff member 710 will the move the severing device 216 from the loaded state 300 to the deployed state 500. For example, the distal portion 708 of the sheath 704 can be proximally retracted, such as for example after proximally retracting the proximal portion 706 of the sheath 704, past the recessed surface 324 of the severing device 216. This will then allow the severing device 216 (e.g., as a result of the biasing force of the spring 308) to move from the loaded state 300 to the deployed state 500.

In further aspects, when the severing device 216 is in the deployed state 500 (as shown in FIG. 9), the distal portion 708 of the sheath 704 can be distally advanced with a distal force from the stiff member 710 (e.g., by activating the first actuator 1110 and/or a different actuator). As the distal portion 708 of the sheath 704 is advanced back over top of the recessed surface 324 of the severing device 216, the distal portion 708 of the sheath 704 will apply a force in a direction towards the shaft 1102 to overcome a force of the spring 308. In some such aspects, the severing device 216 will move from the deployed state 500 to the loaded state 300. In some further aspects, after the severing device 216 has been pivoted from the deployed state 500 to the loaded state 300, the distal portion 708 of the sheath 704 can again be proximally retracted to once again pivot the severing device 216 from the loaded state 300 back to the deployed state 500. For example, a clinician may need to move the severing device 216 from the deployed state 500 to the loaded state 300 temporarily, such as for example, to readjust the transcatheter device 102 and/or change to a different leaflet to be lacerated. In some such examples, the clinician can then move the severing device 216 back to the deployed state 500 and continue utilizing the lacerating device as previously described to lacerate more leaflets. It should be understood this is merely one exemplary reason to move the severing device 216 from the deployed state 500 to the loaded state 300 and then back to the deployed state 500, there may be many other reasons a clinician may need to move the severing device 216 as described and is thus not meant to be limiting. It should be understood that the severing device 216 can be moved (e.g., pivoted) between the loaded state 300 and the deployed state 500 as many times as required, as described herein.

In some aspects, the stiff member 710 can be coupled along any length of the distal portion 708 of the sheath 704. For example, the stiff member 710 can be coupled to a distal segment and/or a proximal segment of the distal portion 708 of the sheath 704. In some embodiments, stiff member 710 can be coupled to an inner radial surface of the distal portion 708 of the sheath 704.

In some aspects, the distal tip 206 can comprise one or more slots 712 configured to receive a portion of the stiff member 710. For example, in some aspects, the portion of the stiff member 710 can slide within the one or more slots 712 to translate the distal portion 708 of the sheath 704. In some aspects the one or more slots 712 can be formed (e.g., cut out, drilled, and/or molded therein) within a portion of the distal tip 206 (e.g., a proximal portion of the distal tip 206).

In some aspects, as shown in FIG. 8 and 10, the stiff member 710 can be disposed within the shaft 1102, such as for example, to route the stiff member 710 from the first actuator 1110 to the distal tip 206. In some aspects, the stiff member 710 can be disposed within the elongated lumen 328 and configured to translate therein (e.g., proximally and/or distally translate therein). In some aspects, the shaft 1102 can comprise an inner shaft 1120 and an outer shaft 1122. In some such aspects, the outer shaft 1122 can comprise one or more passageways 404 that extend in a longitudinal direction of the transcatheter device 102. The one or more passageways 404 can be configured to receive the stiff member 710 therein (e.g., slidably receive the stiff member 710 therein). In some aspects, the stiff member 710 can be disposed between the inner shaft 1120 and the outer shaft 1122. Such as for example, by leaving a gap between at least a portion of the inner shaft 1120 and the outer shaft 1122 to route the stiff member 710 therethrough. In other examples not shown, the inner shaft 1120 can comprise one or more passageways 404 that extend in a longitudinal direction of the transcatheter device 102. The one or more passageways 404 can be configured to receive the stiff member 710 therein (e.g., slidably receive the stiff member 710 therein). In yet another aspect, the shaft 1102 can comprise only a single shaft (e.g., such as only including the inner shaft 1120). In some such aspects, the single shaft can comprise one or more passageways 404 that extend in a longitudinal direction of the transcatheter device 102. The one or more passageways 404 can be configured to receive the stiff member 710 therein (e.g., slidably receive the stiff member 710 therein).

It should be understood that while the stiff member 710 has been described as being routed through/within the shaft 1102 and sliding within the one or more slots 712 of the distal tip 206, the stiff member 710 should not be limited to these embodiments. Any other suitable means of connecting the stiff member 710 from the distal portion 708 of the sheath 704 to the first actuator 1110 may be utilized, such as for example, by routing the stiff member 710 from the distal portion 708 of the sheath 704 to the outer radial surface 214 of the distal tip 206 and along the sheath 704 to be coupled to the first actuator 1110.

In aspects, the stiff member 710 can be a variety of suitable materials. For example, the stiff member 710 can be a material with a relatively high Young's modulus, such as for example, alloys and/or a variety of other metals. The tether 332 material should by no means be limited to the above-mentioned materials and can be any other suitable material to move the severing device 216 between the loaded state 300 and the deployed state 500.

In further aspects, the distal end portion 202, 702 of the transcatheter device 102 can comprise one or more radiopaque markers (e.g., one radiopaque marker, two radiopaque markers, three radiopaque markers, etc.). For example, the one or more radiopaque markers can optionally be disposed along a length of the severing device 216. Additionally and/or alternatively, in some aspects, the one or more radiopaque markers can be disposed along the proximal portion 302 of the severing device 216. Additionally and/or alternatively, the one or more radiopaque markers can be disposed along a length of the distal portion 303 of the severing device 216. Additionally and/or alternatively, the one or more radiopaque markers can be disposed on the distal tip 206 of the transcatheter device 102. For example, the one or more radiopaque markers can be disposed anywhere around the outer radial surface 214 of the distal tip 206. In some aspects, any radiopaque material known in the art may be used for the one or more radiopaque markers. For example, gold, platinum, platinum/iridium, titanium, tantalum, barium silicate, tungsten and/or the like may be utilized for the one or more radiopaque markers. The one or more radiopaque markers should comprise a material that is discernable from other components of the transcatheter device 102. For example, if titanium is utilized in the severing device 216 and/or the distal tip 206, a material other than titanium should be utilized for the one or more radiopaque markers. In this way, the severing device 216 and/or the distal tip 206 will be able to be located by a clinician (e.g., a surgeon) when maneuvering the transcatheter device 102 within the vasculature of a patient. For example, a clinician can utilize fluoroscopic imaging of the one or more radiopaque markers while maneuvering the transcatheter device 102 within the vasculature of a patient. It should be further realized that any other suitable component of the transcatheter device 102 described previously and/or hereinafter can additionally and/or alternatively be provided with one or more radiopaque markers to facilitate a clinician in locating the component with the one or more radiopaque markers within the vasculature and/or heart valve of a patient.

Turning to FIG. 11, the handle device 1108 and the shaft 1102 that can be employed with the transcatheter device 102 are schematically shown. In aspects, the handle device 1108 can comprise the first actuator 1110 (e.g., a plurality of first actuators), as described previously. In aspects, the first actuator 1110 can be configured to activate any one of the severing device 216 (e.g., by way of the tether 332 or stiff member 710), the sheath 204, 704 and/or the distal tip 206 either independently from one another or in combination. For example (as described above), the first actuator 1110 can move the severing device 216 from the loaded state 300 to the deployed state 500, such as for example, by way of the tether 332 or the stiff member 710. In some examples, as illustrated, the first actuator 1110 can be proximally and/or distally advanced, such as for example, by a clinician. In some examples, proximally advancing (e.g., advancing the first actuator 1110 to the right in FIG. 11) the first actuator 1110 can thereby proximally retract the sheath 204, 704 by way of the shaft 1102 that interconnects the handle device 1108 and the distal tip 206. Accordingly, by proximally retracting the sheath 204, 704, the distal portion 1216 of the sheath 204, 704 can move proximally past the recessed surface 324, and thus the severing device 216 can be moved (e.g., pivoted) from the loaded state 300 to the deployed state 500 (e.g., such as by the spring 308 as described above). In some such examples, the first actuator 1110 can be activated to move the severing device 216 from the deployed state 500 to the loaded state 300, such as for example, by distally advancing the first actuator 1110 (e.g., pushing the first actuator 1110 to the right) to slide the sheath 204, 704 over top of the recessed surface 324.

Additionally and/or alternatively, in some aspects, the first actuator 1110 can be activated to move the severing device 216 from the loaded state to the deployed state 500 by way of the tether 332 (as described previously). For example, by actuating the first actuator 1110 (e.g., by sliding the first actuator 1110 to the left) the tension in the tether 332 can be decreased such that the severing device 216 can be moved from the loaded state 300 to the deployed state 500. In some such examples, the first actuator 1110 can be actuated to move the severing device 216 from the deployed state 500 to the loaded state 300 by increasing tension in the tether 332, such as for example, by moving the first actuator 1110 to the right, thereby applying a downward force to the severing device 216 that overcomes the bias of the spring 308. Additionally and/or alternatively, in some aspects, the first actuator 1110 can be activated to move the severing device 216 from the loaded state to the deployed state 500 by way of the stiff member 710 (as described previously). For example, by actuating the first actuator 1110 (e.g., by sliding the first actuator 1110 to the right) the stiff member 710 can proximally retract the distal portion 708 of the sheath 704 to move the distal portion 708 of the sheath 704 proximally past the recessed surface 324, thereby moving the severing device 216 from the loaded state 300 to the deployed state 500. In some such examples, the first actuator 1110 can be activated to move the severing device 216 from the deployed state 500 to the loaded state 300, such as for example, by distally advancing the stiff member 710 by way of the first actuator 1110 to slide the distal portion 708 of the sheath 704 over top of the recessed surface 324.

In some aspects, the first actuator 1110 can comprise a slot 1111 formed within the handle device 1108. In some such aspects, the first actuator 1110 can slide along a length of the slot 1111 to activate the first actuator 1110. For example, in an embodiment with the tether 332, the first actuator 1110 can maintain tension in the tether 332 by being fixed to the right side (i.e., when viewing the orientation shown in FIG. 11) of the slot 1111. In some such examples, moving the actuator from the right side to the left side (e.g., by sliding the first actuator 1110 to the left), can decreased and/or release tension in the tether 332.

In some examples, the handle device 1108 can comprise a second actuator 1132 (e.g., a plurality of second actuators) configured to translate the distal tip 206 relative to the sheath 204, 704. It should be understood that in this embodiment, the first actuator 1110 (or another actuator) will independently actuate the sheath 204, 704, while the second actuate will independently actuate the distal tip 206. In some aspects, the distal tip 206 can proximally and/or distally advance (e.g., distally slide) the distal tip 206 relative to the sheath 204, 704. In some aspects, as shown, the second actuator 1132 can translate the distal tip 206 relative to the sheath 204, 704 by a turning movement of the second actuator 1132 (e.g., a clinician rotating the second actuator 1132). In other aspects, the second actuator 1132 can be similar to that described above for the first actuator 1110 (e.g., slide within a slot 1111 formed within the handle assembly).

In yet another aspect, the handle device 1108 can further comprise a third actuator 1134 (e.g., a plurality of third actuators) configured to translate the sheath 204, 704 relative the distal tip 206. It should be understood that in this embodiment the first actuator 1110 independently actuates the severing device 216, the second actuator 1132 independently actuates the distal tip 206, and the third actuator 1134 independently actuates the sheath 204, 704. For example, the third actuator 1134 can distally advance and/or proximally retract the sheath 204, 704 relative to the distal tip 206. In some aspects, as shown, the third actuator 1134 can operate similarly as described above for the first actuator 1110 and/or the second actuator 1132 (e.g., slide within a slot 1111 formed within the handle assembly).

It is again emphasized herein, that the terms "first, second, and third" as utilized with reference to actuators, is to denote that an actuator activates a specific component of the transcatheter device 102 and is thus not meant to be limiting as to the number and/or types of actuators that may be utilized with the transcatheter device 102. Thus, in some embodiments, more or less actuators may be employed than described above. In some embodiments, the first actuator 1110 can be viewed as the second and/or the third actuator 1134. In some embodiments, the second actuator 1132 can be viewed as the first actuator 1110 and/or the third actuator 1134. In some embodiments, the third actuator 1134 can be viewed as the first actuator 1110 and/or the second actuator 1132. Thus, in any of the embodiments of the actuators described herein, any one of the first actuator 1110, the second actuator 1132 and/or the third actuator 1134 can actuate the same or different components than any other one of the first actuator 1110, the second actuator 1132, and/or the third actuator 1134. In further embodiments, any one of the first actuator 1110, the second actuator 1132, and/or the third actuator 1134 can comprise a plurality of actuators that can independently actuate any of the components described herein (i.e., the first actuator 1110, the second actuator 1132, and/or the third actuator 1134 can perform multiple functions).

As utilized herein, the term translate can mean that a particular component of the transcatheter device 102 (e.g., the distal tip 206, the severing device 216, the sheath 204, 704) can move (e.g., proximally move, distally move, vertically move, and/or radially move), advance (e.g., proximally advance, distally advance, vertically advance, and/or radially advance), slide (e.g., proximally slide, distally slide, vertically slide, and/or radially slide), rotate (e.g., rotate from about 0 degrees to about 360 degrees), shift (e.g., proximally shift, distally shift, vertically shift, and/or radially shift) relative to any one of the other components of the transcatheter device 102.

In some aspects, the handle device 1108 can be configured to actuate components of the transcatheter device 102 by any suitable actuating means. For example, the handle device 1108 can actuate components of the transcatheter device 102 by sliding movements (e.g., proximally and/or distally sliding the actuator in a proximal or distal direction), rotational movements (e.g., clockwise and/or counter clockwise rotation), and/or pressure actuated movements (e.g., button presses). In some aspects, the handle device 1108 can be configured to be mechanically actuated, electrically actuated, electromechanically actuated, and/or any other suitable actuating means. It should be understood that the above examples are not meant to be limiting, and any other suitable handle devices may be utilized with the transcatheter device 102 described herein.

In some embodiments the shaft 1102 can comprise one or more shafts that are coupled together to interconnect the handle device 1108 to various components (e.g., the distal tip 206) of the transcatheter device 102. For example, the shaft 1102 can comprise a first shaft 1114 and a second shaft 1116, that can be coupled together by an extension 1118 of the first shaft 1114. In further aspects, the shaft 1102 can comprise a third shaft 1120 (e.g., the inner shaft 1120 in FIGS. 3 and 7) configured to receive the expandable stent frame 1202 in the contracted orientation 400 (e.g., be compressed around in the contracted orientation 400). In some such examples, the third shaft 1120 can be coupled to the distal tip 206. In yet another aspect, a fourth shaft 1122 (e.g., the outer shaft 1122 in FIGS. 3 and 7) can be provided that can be coupled to the second shaft 1116 by coupling element 1124, such as for example, by sliding the coupling element 1124 overtop the second shaft 1116.

In some examples, the shaft 1102 can further comprise a delivery sheath assembly 1126. In aspects, the delivery sheath assembly 1126 can comprise a delivery sheath shaft 1128 coupled to the sheath 204, 704. In some such examples, activating the first actuator 1110, the second actuator 1132, and/or the third actuator 1134 can proximally and/or distally advance the delivery sheath shaft 1128, thereby also distally and/or proximally retracting the sheath 204, 704. As generally described above, in some embodiments proximally retracting the delivery sheath shaft 1128 can move the severing device 216 from the loaded state 300 to the deployed state 500. In some aspects, the shaft 1102 can also comprise outer stability shaft 1130 coupled to the handle device 1108 which can be configured to increase the stability of the shaft 1102. In some such cases, the outer stability shaft 1130 can increase the rigidity of the shaft 1102 when required, such as for example, when maneuvering the transcatheter device 102 through tortious vasculature. However, while the shaft 1102 has been described above as comprising more than one shaft, only one shaft may be necessary for the transcatheter device 102. Additionally, any other suitable shaft may be utilized with the transcatheter device 102.

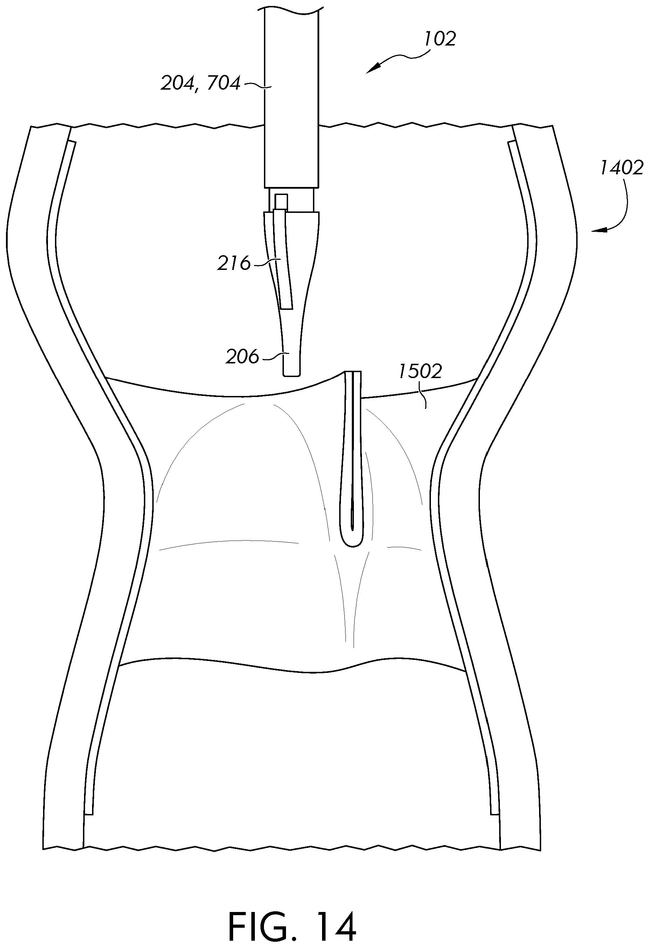

FIGS. 14-21 will now describe a method of replacing a preexisting heart valve 1402 (e.g., a failing heart valve) with initial reference to FIGS. 1-13 with the understanding that similar or identical methods may be provided in the other embodiments of the disclosure. In some aspects, the preexisting heart valve 1402 can comprise an implanted prosthetic heart valve (e.g., a failing implanted prosthetic heart valve), as shown in FIGS. 14-21. In some aspects, the preexisting heart valve 1402 can comprise a native heart valve (e.g., a failing native heart valve), such as for example, a tricuspid valve, a pulmonary valve, a mitral valve, and/or an aortic valve.

Referring to FIG. 14, the method can comprise distally advancing the expandable stent frame 1202 of the transcatheter device 102 in a contracted orientation 400 (see FIG. 4) into the preexisting heart valve 1402. In some aspects, distally advancing the expandable stent frame 1202 can comprise distally advancing the transcatheter device 102. In some examples, distally advancing the transcatheter device 102 can comprise distally advancing the transcatheter device 102 along a guide wire (not shown) through an incision that is made at an anatomical location of the patient's vessel. For example, a transfemoral approach that involves making an incision in the groin and operates as a passageway for guiding the transcatheter device 102 into the preexisting heart valve 1402. In some aspects, the transcatheter device 102 can be distally advanced along the guide wire by way of the elongated lumen 328 extending through the transcatheter device 102. In some aspects, distally advancing the expandable stent frame 1202 can comprise distally advancing a portion of the transcatheter device 102, such as for example, distally advancing a distal portion of the transcatheter device 102. In some further aspects, distally advancing the expandable stent frame 1202 can comprise distally advancing any one of a sheath 204, 704, a shaft 1102 (e.g., an inner shaft 1120 and/or an outer shaft 1122), a handle device 1108, a distal tip 206, and/or any other suitable component of the transcatheter device 102.

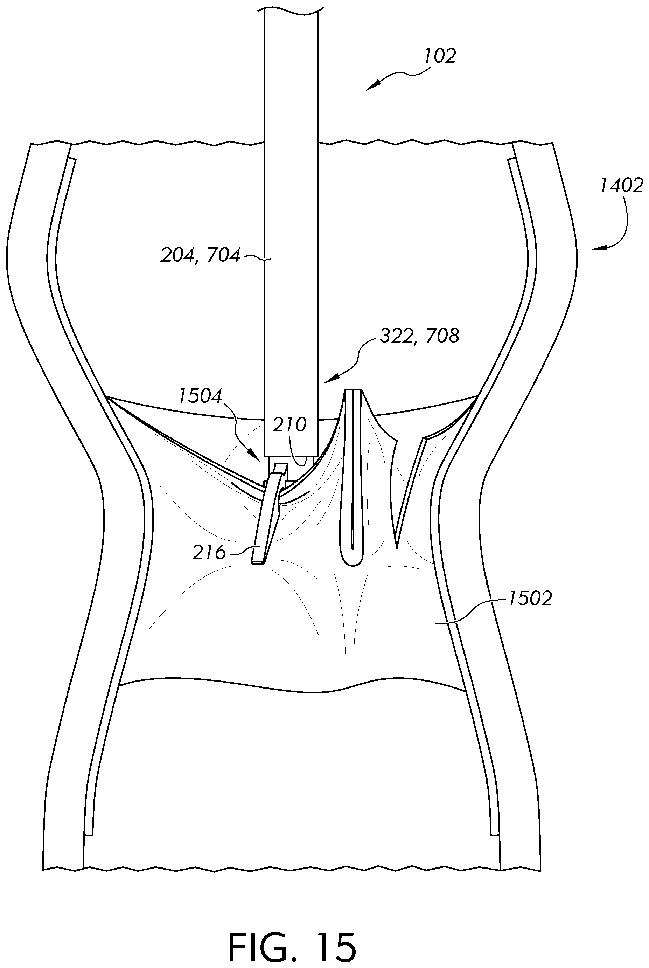

As shown in FIG. 15, the method can further comprise, aligning a portion 1504 of the transcatheter device 102 with leaflets 1502 of the preexisting heart valve 1402. In some examples, the leaflets 1502 can comprise prosthetic heart valve leaflets. For example, the leaflets 1502 can comprise one or more leaflets, such as for example, two leaflets, three leaflets, etc. In some examples, the leaflets 1502 can comprise native heart valve leaflets. For example, the leaflets 1502 can comprise tricuspid valve leaflets, pulmonary valve leaflets, mitral valve leaflets, and/or aortic valve leaflets.

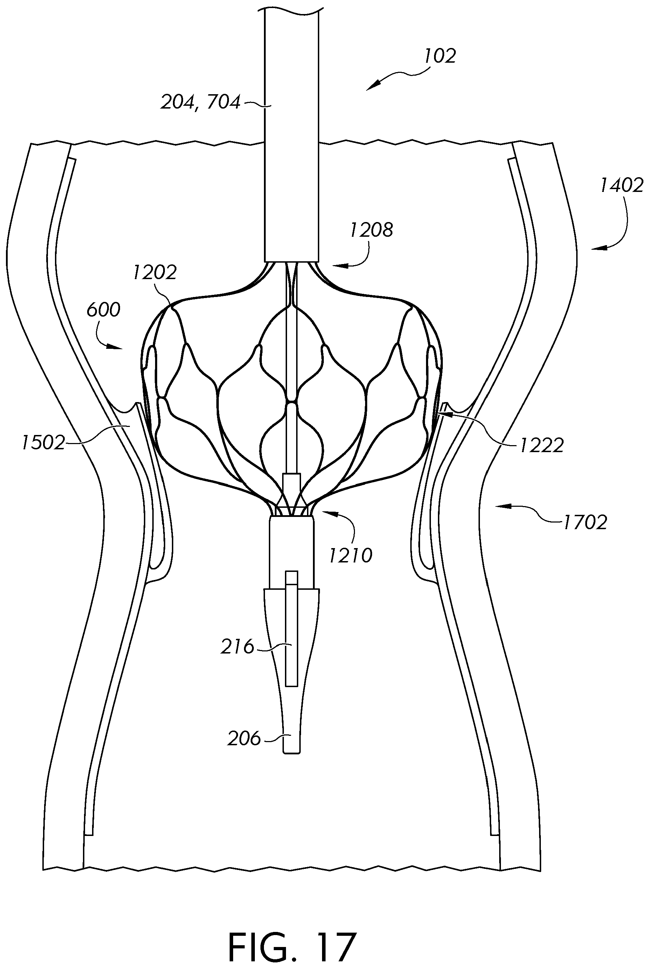

In some aspects, as shown best in FIG. 17, aligning the portion 1504 of the transcatheter device 102 can comprise expanding the expandable stent frame 1202 from the contracted orientation 400 to a partially expanded orientation 600. In further aspects, an intermediate portion 1222 of the expandable stent frame 1202 defined between a proximal end 1208 of the expandable stent frame 1202 and a distal end 1210 of the expandable stent frame 1202 bends radially outward relative to the proximal end 1208 and the distal end 1210. For example, as shown in FIG. 17, the intermediate portion 1222 can radially expand outward within the preexisting heart valve 1402, such that a distance between the intermediate portion 1222 and the preexisting heart valve 1402 is decreased. In some examples, when the distance is decreased, one or more sections of the intermediate portion 1222 can contact one or more sections of the preexisting heart valve 1402. For example, a section of the intermediate portion 1222 can contact the leaflets 1502 of the preexisting heart valve 1402. In some examples, the leaflets 1502 that may be contacted by the intermediate portion 1222 can be the prosthetic heart valve leaflets of an implanted prosthetic heart valve. In other examples, the leaflets that may be contacted by the intermediate portion can be the leaflets of the native heart valve.

In alternative examples, the intermediate portion 1222 can bend radially outward, but not contact the preexisting heart valve 1402. For example, an apex (e.g., the portion that is radially bent outward the furthest) of the intermediate portion 1222 can be positioned to expand above an intermediate portion 1702 of the preexisting heart valve 1402, such that a point of contact (e.g., the apex of the intermediate portion 1222 and the intermediate portion 1702 of the preexisting heart valve 1402 with the smallest diameter) between the intermediate portion 1222 of the expandable stent frame 1202 and the intermediate portion 1702 of the preexisting heart valve 1402 is removed. In this way, the intermediate portion 1222 of the expandable stent frame 1202 will not contact the preexisting heart valve 1402. In some examples where the preexisting heart valve 1402 is an implanted prosthetic heart valve, the intermediate portion 1702 of the preexisting heart valve 1402 can be the intermediate portion 1222 of the implanted prosthetic heart valve. In some examples where the preexisting heart valve 1402 is a native heart valve, the intermediate portion 1702 of the preexisting heart valve 1402 can be an intermediate portion of the native heart valve.

In some aspects, partially expanding the expandable stent frame 1202 further comprises maintaining both the proximal end 1208 (shown generally) and the distal end 1210 (shown generally) in a collapsed state (as shown in FIG. 17). In some examples, partially expanding the expandable stent frame 1202 can comprise proximally retracting a sheath 204, 704 of the transcatheter device 102. In this example, the sheath 204, 704 can be proximally retracted far enough to expand the expandable stent frame 1202 from the contracted orientation 400 to the partially expanded orientation 600, but not so far as to release the proximal end 1208 of the expandable stent frame 1202 from the sheath 204, 704.

Turning back to FIG. 15, in some aspects, the portion 1504 of the transcatheter device 102 that is aligned with the leaflets 1502 of the preexisting heart valve 1402 can comprise a distal portion of the transcatheter device 102. In some examples, the distal portion can be a distal end 210 of the sheath 204, 704. In other examples, the distal portion can be a portion of a distal tip 206 of the transcatheter device 102, such as for example, a proximal portion or a distal portion of the distal tip 206. In some examples, the portion 1504 can comprise a portion of a severing device 216, such as for example, a proximal portion 302 or a distal portion 303 of the severing device 216. In other examples, the portion 1504 can comprise any other suitable portion of the transcatheter device 102.

In some aspects, aligning the transcatheter device 102 with the leaflets 1502 of the preexisting heart valve 1402 can further comprise rotating the severing device 216 about an elongated axis of the transcatheter device 102. For example, the method can comprise rotating the severing device 216 anywhere from about 0 degrees to about 360 degrees about the elongated axis, such as for example, to align the transcatheter device 102 with a portion of the leaflets 1502 to be severed.

In some aspects, aligning the portion 1504 of the transcatheter device 102 can further comprise locating one or more radiopaque markers disposed on one or more portions of the transcatheter device 102 to determine a position of the transcatheter device 102 relative to the preexisting heart valve 1402 and/or the leaflets 1502 of the preexisting heart valve 1402. In some aspects, the one or more radiopaque markers can be located utilizing fluoroscopic imaging. In some aspects, the one or more radiopaque markers can be positioned on the expandable stent frame 1202, and the method can further comprise locating the one or more radiopaque markers positioned on the expandable stent frame 1202 to determine a position of the expandable stent frame 1202 relative to the preexisting heart valve 1402 and/or the leaflets 1502. Additionally and/or alternatively, the one or more radiopaque markers can be positioned on the distal tip 206 of the transcatheter device 102, and the method can further comprise locating the one or more radiopaque markers positioned on the distal tip 206 of the transcatheter device 102 to determine a position of the distal tip 206 relative to the preexisting heart valve 1402 and/or the leaflets 1502 of the preexisting heart valve 1402. Additionally and/or alternatively, the one or more radiopaque markers can be positioned on the severing device 216 of the transcatheter device 102, and the method can further comprise locating the one or more radiopaque markers positioned the severing device 216 to determine a position of the severing device 216 relative to the preexisting heart valve 1402 and/or the leaflets 1502 of the preexisting heart valve 1402.

In some aspects, as shown in FIG. 15, the method can comprise moving the severing device 216 of the transcatheter device 102 from a loaded state 300 to a deployed state 500. In aspects, the severing device 216 can be moved from the loaded state 300 to the deployed state 500 after locating the one or more radiopaque markers. In some aspects, moving the severing device 216 can comprise pivoting the severing device 216 from the loaded state 300 to the deployed state 500. In some aspects, the method can further comprise proximally retracting the sheath 204, 704 to pivot the severing device 216 from the loaded state 300 to the deployed state 500. In further aspects, moving the severing device 216 from the loaded state 300 to the deployed state 500 can comprise proximally retracting a distal portion 322, 708 of the sheath 204, 704. In some aspects, moving the severing device 216 from the loaded state 300 to the deployed state 500 can comprise releasing tension in a tether 332. In some aspects, the severing device 216 can be moved from the loaded state 300 to the deployed state 500 after expanding the expandable stent frame 1202 from the contracted orientation 400 to the partially expanded orientation 600. In some such aspects, the severing device 216 can be moved from the loaded state 300 to the deployed state 500 after expanding the expandable stent frame 1202 from the contracted orientation 400 to the partially expanded orientation 600 by releasing tension in the tether 332 (see FIGS. 3 and 5) or by proximally advancing a stiff member 710 to proximally advance the distal portion 708 of the sheath 704 (see Figs. 7 and 9).

In some aspects, the severing device 216 can be moved from the loaded state 300 to the deployed state 500 while simultaneously expanding the expandable stent frame 1202 from the contracted orientation 400 to the partially expanded orientation 600.

In some aspects, the method can further comprise moving the severing device 216 from the deployed state 500 to the loaded state 300. In some aspects, moving the severing device 216 from the deployed state 500 to the loaded state 300 can comprise placing the tether 332 under tension. In further aspects, moving the severing device 216 from the deployed state 500 to the loaded state 300 can further comprise distally advancing the sheath 204, 704. In some such aspects, moving the severing device 216 from the deployed state 500 to the loaded state 300 can comprise moving a distal portion 322, 708 of the sheath 204, 704. In some aspects, the severing device 216 can be moved from the deployed state 500 to the loaded state 300 after expanding the expandable stent frame 1202 from the contracted orientation 400 to the partially expanded orientation 600. In some such aspects, the severing device 216 can be moved from the deployed state 500 to the loaded state 300 after expanding the expandable stent frame 1202 from the contracted orientation 400 to the partially expanded orientation 600 by placing the tether 332 under tension (see FIGS. 3 and 5) or by distally advancing a stiff member 710 to distally advance the distal portion 708 of the sheath 704 (see Figs. 7 and 9).

In aspects, the method can further comprise energizing a portion of the severing device 216 after moving the severing device 216 from the loaded state 300 to the deployed state 500. In some examples, the portion of the severing device 216 that is energized comprises a spring 308. In some examples, the method can comprise moving the severing device 216 from the loaded state 300 to the deployed state 500 with the spring 308, and then the method can further comprise energizing the portion of the spring 308.

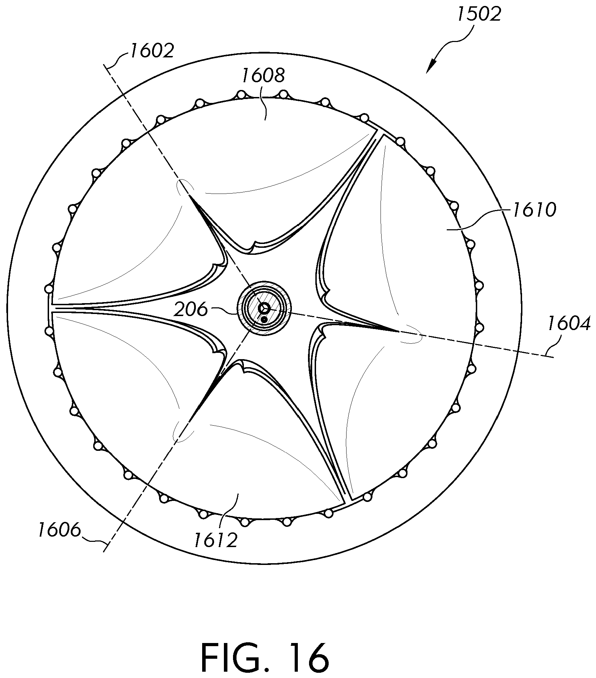

Turning to FIGS. 16 and 17, the method can comprise severing at least one of the leaflets 1502 with the severing device 216 in the deployed state 500. In some aspects, severing at least one of the leaflets 1502 can comprise distally advancing the transcatheter device 102 after moving the severing device 216 from the loaded state 300 to the deployed state 500 and energizing the portion of the severing device 216. In some aspects, severing at least one of the leaflets 1502 can comprise contacting at least one of the leaflets 1502 with the energized portion of the severing device 216. For example, the energized portion can comprise the spring and contacting at least one of the leaflets 1502 can comprise contacting at least one of the leaflets 1502 with a region 316 of the spring.

In some aspects, severing at least one of the leaflets 1502 can comprise cauterizing at least one of the leaflets 1502 along a length of at least one of the leaflets 1502. For example, a dashed line 1602, 1604, 1606, as shown in FIG. 16, represents a general line that may be followed to cauterize at least one of the leaflets 1502. In some such examples, at least one of the leaflets 1502 can be cauterized from its free end in a direction along the dashed line 1602, 1604, 1606 to an attached end of the leaflets 1502. In some aspects, as shown best in FIG. 16, severing at least one of the leaflets 1502 can further comprise severing a first leaflet 1608, a second leaflet 1610, and/or a third leaflet 1612.

In some aspects, the method can further comprise rotating the severing device 216 from any one of the first leaflet 1608, the second leaflet 1610, and/or the third leaflet 1612 after first severing any one of the first leaflet 1608, the second leaflet 1610, and/or the third leaflet 1612. For example, while viewing FIG. 16, the method can first comprise severing (e.g., cauterizing) the first leaflet 1608 (e.g., along the dashed line 1602), then rotating the transcatheter device 102 about the elongated axis of the transcatheter device 102 about one third of a revolution (e.g., one third of 360 degrees, or about 120 degrees) clockwise to align with the second leaflet 1610. The method can then comprise severing the second leaflet 1610 along the dashed line 1604. After this, the method can then comprise rotating the transcatheter device 102 about the elongated axis of the transcatheter device 102 about another one third of a revolution (e.g., one third of 360 degrees, or about 120 degrees) clockwise to align with the third leaflet 1612. The method can then comprise severing the third leaflet 1612 along the dashed line 1606. It should be understood that this example is merely exemplary, and thus any combination of ways to sever the leaflets 1502 may be employed. For example, the transcatheter device 102 can be rotated clockwise and/or counter clockwise, the transcatheter device 102 can be rotated any number of degrees or revolutions, such as for example, greater than or less than 120 degrees. While the method above has been described as severing three leaflets 1502, any other number of leaflets 1502 may be severed (e.g., less than three leaflets 1502).

In some aspects, severing at least one of the leaflets 1502 can further comprise bisecting at least one of the leaflets 1502. For example, bisecting at least one of the leaflets 1502 can comprise lacerating at least one of the leaflets 1502 to divide the at least one of the leaflets 1502 into two substantially equal parts, such as for example, as generally indicated by the dashed line 1602, 1604, 1606 in FIG. 16. In some aspects, the method can comprise moving the severing device 216 from the deployed state 500 to the loaded state 300 after severing one of the leaflets 1502. In some non-limiting examples, the method can comprise moving the severing device 216 from the deployed state 500 to the loaded state 300 after severing any one of the first leaflet 1608, the second leaflet 1610, and/or the third leaflet 1612 but before severing anyone other one of the first leaflet 1608, the second leaflet 1610 and/or the third leaflet 1612. In some examples, the method can comprise maintaining the severing device 216 in the deployed state 500 for the entire duration of severing any one of the leaflets 1502 (e.g., the first leaflet 1608, the second leaflet 1610, and/or the third leaflet 1612).

It should be understood that while the expandable stent frame 1202 has been discussed above as being expanded from the contracted orientation 400 to the partially expanded orientation 600 with specific reference to FIG. 15, in other aspects, the severing device 216 can be aligned without partially expanding the expandable stent frame 1202, and thus can be expanded from the contracted orientation 400 to the partially expanded orientation 600 after severing at least one of the leaflets 1502. In yet another aspect, the expandable stent frame 1202 can be expanded from the contracted orientation 400 to the partially expanded orientation 600 while simultaneously severing at least one of the leaflets 1502. In yet another aspect, discussed in more detail below, the expandable stent frame 1202 does not have to be expanded from the contracted orientation 400 to the partially expanded orientation 600 at all.

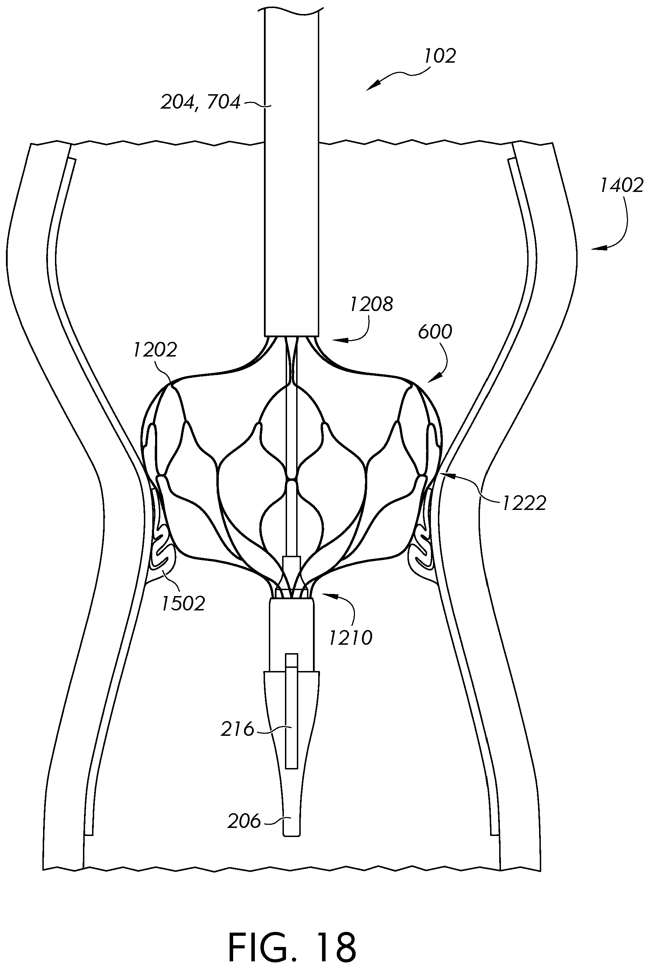

In some aspects, as shown in FIG. 18, the method can further comprise distally advancing the partially expanded stent frame (e.g., the expandable stent frame 1202 in the partially expanded orientation 600) to reposition the leaflets 1502 of the preexisting heart valve 1402. Prior to distally advancing the transcatheter device 102, the partially expanded stent frame may already be in contact with the leaflets 1502 or it may not be in contact with the leaflets 1502 and is brought into contact with the leaflets 1502 when advanced. In some aspects, repositioning the leaflets 1502 can comprise folding, collapsing, flattening, pushing, and/or bunching the leaflets 1502 in any one of a distal direction, a radial direction, and/or a proximal direction within the preexisting heart valve 1402. In further aspects, the leaflets 1502 are repositioned after severing at least one of the leaflets 1502. In some aspects, the leaflets 1502 can be repositioned prior to severing at least one of the leaflets 1502. In yet another aspect, the leaflets 1502 can be repositioned prior to and subsequent to severing at least one of the leaflets 1502.

In some aspects, such as for example, where the expandable stent frame 1202 is expanded from the contracted orientation 400 to the partially expanded orientation 600 while aligning the transcatheter device (as shown in FIG. 15), at least one of the leaflets 1502 can be repositioned at the same time as severing at least one of the leaflets 1502. For example, when at least one of the leaflets 1502 is severed, (see FIG. 17) the partially expanded stent frame can also simultaneously reposition at least one of the leaflets 1502 (e.g., the leaflet that was just severed, other severed leaflets, and/or other unsevered leaflets).

In some aspects, after expanding the expandable stent frame 1202 from the contracted orientation 400 to the partially expanded orientation 600, the method can further comprise recapturing the expandable stent frame 1202 to collapse the expandable stent frame 1202 from the partially expanded orientation 600 to the contracted orientation 400 prior to expanding the expandable stent frame 1202 from the partially expanded orientation 600 to a fully expanded orientation 1200. In some particular embodiments, recapturing the expandable stent frame 1202 can comprise distally advancing a distal portion 322 of the sheath 204. In this way, the distal portion 322 of the sheath 204 can radially compress a proximal portion of the intermediate portion 1222 of the expandable stent frame 1202 within the distal portion 322 of the sheath 204. In other particular embodiments, recapturing the expandable stent frame 1202 can comprise distally advancing a proximal portion 706 of the sheath 704. In this way, the proximal portion 706 of the sheath 704 can radially compress a proximal portion of the intermediate portion 1222 of the expandable stent frame 1202 within the proximal portion 706 of the sheath 704. In some such particular examples, recapturing the expandable stent frame 1202 can further comprise proximally retracting a distal portion 708 of the sheath 704. In this way, the distal portion 708 of the sheath 704 can radially compress a distal portion of the intermediate portion 1222 within the distal portion 708 of the sheath 704. In further such particular examples, recapturing the expandable stent frame 1202 can further comprise distally advancing the proximal portion 706 of the sheath 704 while simultaneously proximally retracting the distal portion 708 of the sheath 704.

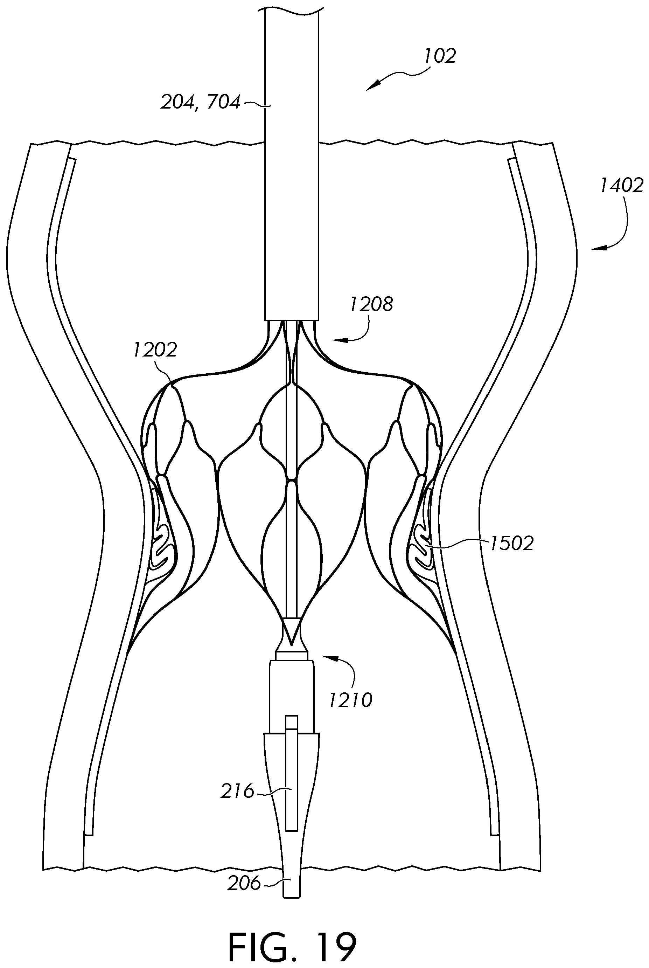

Turning now to FIGS. 19-20, in aspects, the method can comprise radially expanding the expandable stent frame 1202 after severing at least one of the leaflets 1502. In some aspects, radially expanding the expandable stent frame 1202 comprises radially expanding the expandable stent frame 1202 from the partially expanded orientation 600 to the fully expanded orientation 1200 by releasing the proximal end 1208 to radially expand and releasing the distal end 1210 to radially expand.

Turning to FIG. 19, in some aspects, radially expanding the expandable stent frame 1202 from the partially expanded orientation 600 to the fully expanded orientation 1200 comprises initially releasing the distal end 1210 to radially expand prior to releasing the proximal end 1208. In some such aspects, the distal end 1210 can achieve a radially expanded state while the proximal end 1208 is maintained in the collapsed state. In some aspects, releasing the distal end 1210 can comprise distally advancing the distal tip 206 of the transcatheter device 102 relative to the expandable stent frame 1202. In some particular embodiments (such as the embodiments shown in FIGS. 7-10), releasing the distal end 1210 can comprise distally advancing the distal portion 708 of the sheath 704 relative to the expandable stent frame 1202.

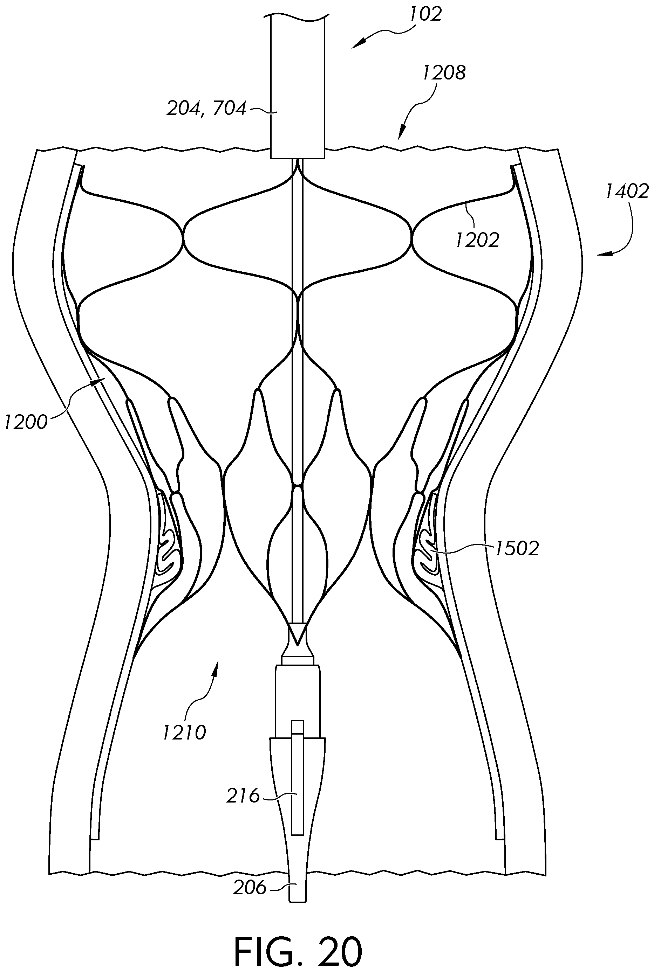

In further aspects, as shown in FIG. 20, radially expanding the expandable stent frame 1202 from the partially expanded orientation 600 to the fully expanded orientation 1200 can further comprise releasing the proximal end 1208 to radially expand after initially releasing the distal end 1210. In some aspects, releasing the proximal end 1208 can comprise proximally retracting the sheath 204, 704 of the transcatheter device 102 relative to the expandable stent frame 1202. In some non-limiting examples, releasing the proximal end 1208 can comprise proximally retracting the proximal portion 402, 706 of the sheath 204, 704.

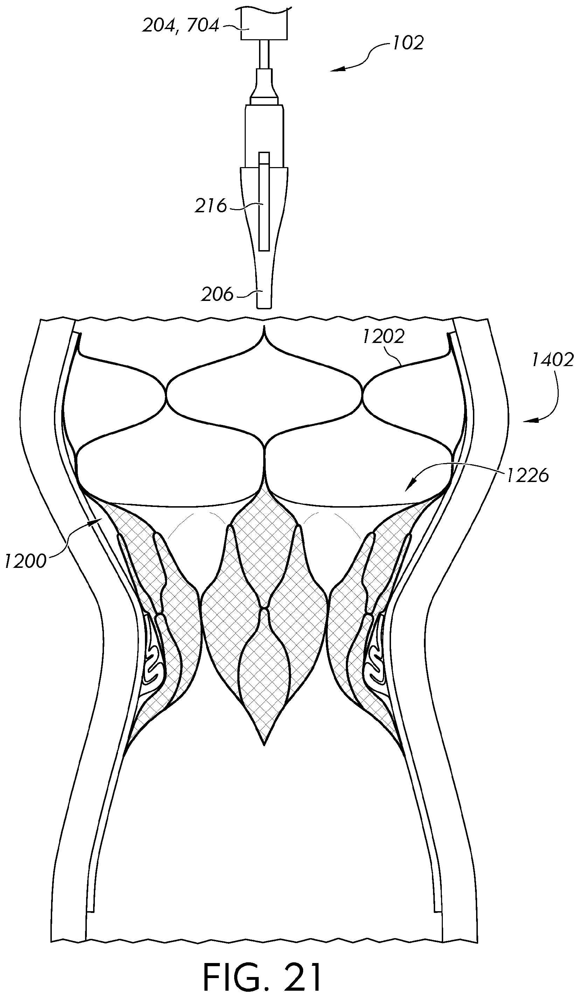

In some aspects, radially expanding the expandable stent frame 1202 can further comprise radially expanding the expandable stent frame 1202 from the contracted orientation 400 to the fully expanded orientation 1200 (as shown in FIG. 20 and 21). For example, in some embodiments the transcatheter device 102 can be employed without expanding the expandable stent frame 1202 from the contracted orientation 400 to the partially expanded orientation 600, such as for example, when the leaflets 1502 do not need to be repositioned (e.g., the leaflets 1502 will not obstruct the coronary arteries). In some such examples, the expandable stent frame 1202 can be expanded directly from the contracted orientation 400 to the fully expanded orientation 1200.

In some aspects (as shown in FIG. 21) the transcatheter device 102 can further comprise a prosthetic heart valve 1226 comprising the expandable stent frame 1202. In some such aspects, expanding the expandable stent frame 1202 can further comprise deploying a valve structure. In some aspects, the valve structure can comprise one or more leaflets 1228 (e.g., one or more leaflets that function similar to the leaflets 1502 being replaced). In some examples, expanding the expandable stent frame 1202 can further comprise inflating an expansion device (not shown) positioned within an inner lumen 1204 of the expandable stent frame 1202. In some aspects, the expandable stent frame 1202 is radially expanded from the partially expanded orientation 600 to the fully expanded orientation 1200 after severing at least one of the leaflets 1502 and repositioning at least one of the severed leaflets 1502 with the partially expanded stent frame. In some aspects, the repositioned leaflets 1502 are pinned in place relative to the expandable frame.

In some examples, the leaflets 1502 are radially pinned against the preexisting heart valve 1402, such that they are prohibited from movement (e.g., radial, distal and/or proximal movement). This may be beneficial in preventing obstruction by the leaflets 1502, such as for example, by preventing the left and right coronary arteries from being obstructed by the leaflets 1502 while replacing the preexisting heart valve 1402. In some examples, expanding the expandable stent frame 1202 can further comprise indexing the expandable stent frame 1202 with the preexisting heart valve 1402. In some examples, "index" can mean contacting, engaging, holding, interlocking, interconnecting, joining, mating, coupling, and/or the like.

Turning to FIG. 21, in some aspects, the method can comprise removing the transcatheter device 102 from the preexisting heart valve 1402. For example, the method can comprise proximally retracting the transcatheter device 102 to remove the transcatheter device 102 from the preexisting heart valve 1402 and out of the patient's vasculature. In some examples, removing the transcatheter device 102 from the preexisting heart valve 1402 can first comprise proximally retracting the distal tip 206, such as for example, proximally retracting the distal tip 206 to abut the distal end 210 of the sheath 204, 704, and then further comprising removing the transcatheter device 102 from the preexisting heart valve 1402. In other examples, removing the transcatheter device 102 from the preexisting heart valve 1402 can comprise proximally retracting the distal tip 206, thereby removing the transcatheter device 102 from the preexisting heart valve 1402. In this example, the transcatheter device 102 can then be proximally retracted from the patient's vasculature. In some examples where the transcatheter device 102 comprises an expansion device, the method can further comprise deflating the expansion device (not shown), and then removing the transcatheter device 102 from the preexisting heart valve 1402.

In accordance with the disclosure, non-limiting aspects of the disclosure will now be described. Various combinations of the aspects can be provided in accordance with the disclosure.

Aspect 1. A transcatheter device comprises a shaft comprising a proximal portion and a distal portion. The transcatheter device further comprises a handle device coupled to the proximal portion of the shaft, wherein the handle device comprises a first actuator. The transcatheter device further comprises a distal tip coupled to the distal portion of the shaft. The transcatheter device further comprises a sheath disposed around the shaft and configured to translate relative to the distal tip. The transcatheter device further comprises a severing device comprising a proximal portion pivotally mounted to the distal tip and configured to be pivoted from a loaded state to a deployed state. The transcatheter device further comprises a first actuator configured to be activated to move the severing device from the loaded state to the deployed state.

Aspect 2. The transcatheter device of Aspect 1, wherein the severing device further comprises a spring configured to bias the severing device to the deployed state.

Aspect 3. The transcatheter device of Aspect 2, wherein the spring comprises a region that is configured to be energized in the deployed state.

Aspect 4. The transcatheter device of any one of Aspects 1 - 3, wherein the first actuator comprises a tether configured to pivot the severing device from the deployed state to the loaded state.

Aspect 5. The transcatheter device of any one of Aspects 1 - 4, further comprising an expandable stent frame, the expandable stent frame comprising a proximal end and a distal end, wherein the proximal end is configured to be received within the sheath, and wherein the expandable stent frame is configured to achieve a contracted orientation, a partially expanded orientation, and a fully expanded orientation.

Aspect 6. The transcatheter device of Aspect 5, wherein an intermediate portion of the expandable stent frame defined between the proximal end and the distal end is configured to bend radially outward in the partially expanded orientation.

Aspect 7. The transcatheter device of Aspect 6, wherein the partially expanded orientation comprises the intermediate portion being bent radially outward relative to the sheath.

Aspect 8. The transcatheter device of any one of Aspects 5 - 7, wherein the contracted orientation comprises the proximal end of the expandable stent frame being in a collapsed state within the sheath.

Aspect 9. The transcatheter device of any one of Aspects 5 - 8, wherein the contracted orientation further comprises the distal end of the expandable stent frame being in the collapsed state within the distal tip.

Aspect 10. The transcatheter device of any one of Aspects 5 - 9, wherein the partially expanded orientation comprises the proximal end of the expandable stent frame being in a collapsed state within the sheath.

Aspect 11. The transcatheter device of any one of Aspects 5 - 10, wherein the partially expanded orientation further comprises the distal end of the expandable stent frame being in the collapsed state within the distal tip.

Aspect 12. The transcatheter device of any one of Aspects 5 - 11, further comprising a prosthetic heart valve comprising the expandable stent frame.

Aspect 13. The transcatheter device of any one of Aspects 1 - 12, wherein the handle device comprises a second actuator configured to translate the distal tip relative to the sheath.

Aspect 14. The transcatheter device of any one of Aspects 1 - 13, wherein the handle device further comprises a third actuator configured to translate the sheath relative the distal tip.

Aspect 15. The transcatheter device of any one of Aspects 1 - 14, wherein the severing device is pivotally mounted within a recess of the distal tip.

Aspect 16. The transcatheter device of Aspect 15, wherein the recess is configured to receive the severing device in the loaded state, and further wherein an outer radial surface of the severing device contours to an outer radial surface of the distal tip to define a flush surface when the severing device is received within the recess in the loaded state.

Aspect 17. The transcatheter device of any one of Aspects 1 - 16, wherein the sheath comprises a proximal portion and a distal portion.

Aspect 18. The transcatheter device of Aspect 17, wherein the proximal portion and the distal portion of the sheath are configured to translate relative to one another.

Aspect 19. The transcatheter device of any one of Aspects 17 - 18, wherein the loaded state comprises the distal portion of the sheath disposed around the proximal portion of the severing device.

Aspect 20. A method of replacing a heart valve comprises distally advancing an expandable stent frame of a transcatheter device in a contracted orientation into the heart valve. The method further comprises, aligning a portion of the transcatheter device with leaflets of the heart valve. The method further comprises moving a severing device of the transcatheter device from a loaded state to a deployed state. The method further comprises severing at least one of the leaflets with the severing device in the deployed state. The method further comprises radially expanding the expandable stent frame after severing at least one of the leaflets.

Aspect 21. The method of Aspect 20, wherein moving the severing device comprises pivoting the severing device from the loaded state to the deployed state.

Aspect 22. The method of Aspect 21, further comprising proximally retracting a sheath to pivot the severing device from the loaded state to the deployed state.

Aspect 23. The method of any one of Aspects 20 - 22, wherein aligning the portion of the transcatheter device comprises expanding the expandable stent frame from the contracted orientation to a partially expanded orientation, wherein an intermediate portion of the expandable stent frame defined between a proximal end of the expandable stent frame and a distal end of the expandable stent frame bends radially outward relative to the proximal end and the distal end.

Aspect 24. The method of Aspect 23, wherein partially expanding the expandable stent frame further comprises maintaining both the proximal end and the distal end in a collapsed state.

Aspect 25. The method of any one of Aspects 23 - 24, further comprising distally advancing the partially expanded stent frame to reposition the leaflets of the heart valve.

Aspect 26. The method of Aspect 25, wherein the leaflets are repositioned after severing at least one of the leaflets.

Aspect 27. The method of any one of Aspects 24 - 25, wherein radially expanding the expandable stent frame comprises radially expanding the expandable stent frame from the partially expanded orientation to a fully expanded orientation by releasing the proximal end to radially expand and releasing the distal end to radially expand.

Aspect 28. The method of Aspect 27, wherein radially expanding the expandable stent frame from the partially expanded orientation to the fully expanded orientation comprises initially releasing the distal end to radially expand prior to releasing the proximal end, wherein the distal end achieves a radially expanded state while the proximal end is maintained in the collapsed state.

Aspect 29. The method of Aspect 28, wherein radially expanding the expandable stent frame from the partially expanded orientation to the fully expanded orientation further comprises releasing the proximal end to radially expand after initially releasing the distal end.

Aspect 30. The method of any one of Aspects 27 - 29, wherein the expandable stent frame is radially expanded from the partially expanded orientation to the fully expanded orientation after severing at least one of the leaflets and repositioning at least one of the severed leaflets with the partially expanded stent frame.

Aspect 31. The method of any one of Aspects 27 - 30, wherein releasing the distal end comprises distally advancing a distal tip of the transcatheter device relative to the expandable stent frame.

Aspect 32. The method of any one of Aspects 27 - 30, wherein releasing the proximal end comprises proximally retracting a sheath of the transcatheter device relative to the expandable stent frame.

Aspect 33. The method of any one of Aspects 27 - 32, wherein, after expanding the expandable stent frame from the contracted orientation to the partially expanded orientation, further comprising recapturing the expandable stent frame to collapse the expandable stent frame from the partially expanded orientation to the contracted orientation prior to expanding the expandable stent frame from the partially expanded orientation to the fully expanded orientation.

Aspect 34. The method of any one of Aspects 20 - 33, further comprising moving the severing device from the deployed state to the loaded state.

Aspect 35. The method of Aspect 34, wherein moving the severing device from the deployed state to the loaded state comprises placing a tether under tension.

Aspect 36. The method of Aspect 34, wherein moving the severing device from the deployed state to the loaded state further comprises distally advancing a sheath.

Aspect 37. The method of any one of Aspects 34 - 36, wherein the severing device is moved from the deployed state to the loaded state after expanding the expandable stent frame.

Aspect 38. The method of any one of Aspects 20 - 37, further comprising energizing a portion of the severing device after moving the severing device from the loaded state to the deployed state.

Aspect 39. The method of Aspect 38, wherein the portion of the severing device that is energized comprises a spring.

Aspect 40. The method of any one of Aspects 38 - 39, wherein severing at least one of the leaflets comprises contacting at least one of the leaflets with the energized portion of the severing device.

Aspect 41. The method of any one of Aspects 20 - 40, wherein severing at least one of the leaflets comprises cauterizing at least one of the leaflets along a length of at least one of the leaflets.

Aspect 42. The method of any one of Aspects 20 - 41, wherein severing at least one of the leaflets further comprises severing a first leaflet, a second leaflet, and/or a third leaflet.

Aspect 43. The method of Aspect 42, further comprises rotating the severing device from any one of the first leaflet, the second leaflet, and/or the third leaflet after first severing any one of the first leaflet, the second leaflet, and/or the third leaflet.

Aspect 44. The method of any one of Aspects 20 - 43, wherein the portion of the transcatheter device that is aligned with the leaflets of the heart valve comprises a distal portion of the transcatheter device.

Aspect 45. The method of any one of Aspects 20 - 44, wherein aligning the transcatheter device with the leaflets of the heart valve further comprises rotating the severing device about an elongated axis of the transcatheter device.

Aspect 46. The method of any one of Aspects 20 - 45, wherein severing at least one of the leaflets further comprises bisecting at least one of the leaflets.

Aspect 47. The method of any one of Aspects 20 - 46, further comprising a prosthetic heart valve comprising the expandable stent frame.

Aspect 48. The method of any one of Aspects 20 - 47, wherein the heart valve comprises an implanted prosthetic heart valve.

## Claims

1. A transcatheter device (102) comprising:
a shaft (1102) comprising a proximal portion (1104) and a distal portion (1106);
a handle device (1108) coupled to the proximal portion (1104) of the shaft (1102), wherein the handle device (1108) comprises a first actuator (1110);
a distal tip (206) coupled to the distal portion (1106) of the shaft (1102);
a sheath (204, 704) disposed around the shaft (1102) and configured to translate relative to the distal tip (206);
a severing device (216) comprising a proximal portion (302) pivotally mounted to the distal tip (206) and configured to be pivoted from a loaded state (300) to a deployed state (500); and
a first actuator (1110) configured to be activated to move the severing device (216) from the loaded state (300) to the deployed state (500).

2. The transcatheter device (102) of claim 1, wherein the severing device (216) further comprises a spring (308) configured to bias the severing device (216) to the deployed state (500).

3. The transcatheter device (102) of claim 2, wherein the spring (308) comprises a region (316) that is configured to be energized in the deployed state (500).

4. The transcatheter device (102) of any one of claims 1 - 3, further comprising an expandable stent frame (1202), the expandable stent frame (1202) comprising a proximal end (1208) and a distal end (1210), wherein the proximal end (1208) is configured to be received within the sheath (204, 704), and wherein the expandable stent frame (1202) is configured to achieve a contracted orientation (400), a partially expanded orientation (600), and a fully expanded orientation (1200).

5. The transcatheter device (102) of claim 4, further comprising a prosthetic heart valve (1226) comprising the expandable stent frame (1202).

6. The transcatheter device (102) of any one of claims 1 - 5, wherein the severing device (216) is pivotally mounted within a recess (502) of the distal tip (206).

7. The transcatheter device (102) of claim 1, wherein the loaded state (300) comprises a distal portion (1106) of the sheath (204, 704) disposed around the proximal portion (302) of the severing device (216).

8. A method of replacing a heart valve comprising:
distally advancing an expandable stent frame (1202) of a transcatheter device (102) in a contracted orientation (400) into the heart valve;
aligning a portion (1504) of the transcatheter device (102) with leaflets (1228) of the heart valve;
moving a severing device (216) of the transcatheter device (102) from a loaded state (300) to a deployed state (500);
severing at least one of the leaflets (1228) with the severing device (216) in the deployed state (500); and
radially expanding the expandable stent frame (1202) after severing at least one of the leaflets (1228).

9. The method of claim 8, wherein moving the severing device (216) comprises pivoting the severing device (216) from the loaded state (300) to the deployed state (500).

10. The method of claim 9, further comprising proximally retracting a sheath (204, 704) to pivot the severing device (216) from the loaded state (300) to the deployed state (500).

11. The method of any one of claims 8 - 10, wherein aligning the portion (1504) of the transcatheter device (102) comprises expanding the expandable stent frame (1202) from the contracted orientation (400) to a partially expanded orientation (600), wherein an intermediate portion (1222) of the expandable stent frame (1202) defined between a proximal end (1208) of the expandable stent frame (1202) and a distal end (1210) of the expandable stent frame (1202) bends radially outward relative to the proximal end (1208) and the distal end (1210).

12. The method of claim 11, further comprising distally advancing the partially expanded stent frame to reposition the leaflets (1228) of the heart valve.

13. The method of claim 12, wherein radially expanding the expandable stent frame (1202) comprises radially expanding the expandable stent frame (1202) from the partially expanded orientation (600) to a fully expanded orientation (1200) by releasing the proximal end (1208) to radially expand and releasing the distal end (1210) to radially expand.

14. The method of any one of claims 8 - 13, further comprising energizing a portion of the severing device (216) after moving the severing device (216) from the loaded state (300) to the deployed state (500).

15. The method of claim 14, wherein the portion of the severing device (216) that is energized comprises a spring (308).
